# EUROPEAN PATENT APPLICATION

(11) **EP 4 814 118 A1**
(43) Date of publication of application: **30.09.2026**
(21) Application number: 24893463.0
(22) Date of filing: 20.11.2024
(51) Int. Cl.: C12N 15/38, C12N 15/62, C07K 19/00, A61K 39/25, A61P 31/22

(54) **VZV ANTIGEN VARIANT, NUCLEIC ACID, PHARMACEUTICAL COMPOSITION AND USE THEREOF**

(30) Priority: 20.11.2023 CN 202311552335; 16.04.2024 CN 202410460219
(71) Applicant: Shanghai Rnacure Biopharma Co., Ltd., Shanghai 201906 (CN); Walvax Biotechnology Co., Ltd., Kunming, Yunnan 650101 (CN); Yunnan Vaccine Laboratory Co., Ltd., Kunming, Yunnan 650101 (CN)
(72) Inventor: LIN, Jinzhong, Shanghai 201906 (CN); LU, Jing, Shanghai 201906 (CN); YU, Zhaoli, Shanghai 201906 (CN); GU, Hao, Shanghai 201906 (CN); HUANG, Wei, Shanghai 201906 (CN); JIANG, Ting, Shanghai 201906 (CN); CAO, Lin, Shanghai 201906 (CN); TAN, Shudan, Shanghai 201906 (CN)
(74) Representative: Ipsilon Belgium
(86) International application number: PCT/CN2024/133211
(87) International publication number: WO 2025/108306

(57) **Abstract**

The present invention discloses a VZV antigen variant, a nucleic acid, a pharmaceutical composition, and uses thereof. The VZV antigen variant has a Y582G difference compared to the amino acid sequence shown in SEQ ID NO: 1; and/or, the VZV antigen variant has a deletion of positions 561-623, 569-623, or 574-623 compared to the amino acid sequence shown in SEQ ID NO: 1; and/or, the VZV antigen variant has a modification of the protein transmembrane domain and intracellular domain compared to the amino acid sequence shown in SEQ ID NO: 1, wherein the modification of the protein transmembrane domain and intracellular domain comprises replacing the transmembrane domain and intracellular domain of the original VZV antigen with the transmembrane domain of the SARS-CoV-2 Spike protein or the transmembrane domain of the influenza H protein. The VZV antigen variant has stronger immunogenicity than VZV antigens in the prior art, can achieve higher binding antibody titers, and has higher protective efficacy.

## Description

### Cross-Reference to Related Applications

This application claims the priority of Chinese Patent Application No. 202311552335X filed on November 20, 2023 and Chinese Patent Application No. 2024104602193 filed on April 16, 2024. This application cites the full text of the aforementioned Chinese patent applications.

### Technical Field

The invention belongs to the field of vaccines, and specifically relates to a VZV antigen variant, nucleic acid, pharmaceutical composition and application thereof.

### Background

Varicella zoster virus (VZV) belongs to the subfamily Alphaherpesvirinae, namely Human Herpesvirus 3. The viral particle is 150 nm-200 nm in size and is a linear double-stranded DNA virus. Nine glycoproteins are embedded in the viral envelope, namely gE, gI, gC, gH, gL, gB, gK, gM and gN. Among them, gE is the major viral structural protein, a key protein for viral replication and assembly, the most important glycoprotein recognized by the host immune system, and also the main target protein for vaccine design.

Primary infection with VZV usually occurs in early childhood, with chickenpox as the main clinical manifestation. After infection, the virus latently persists in neurons of human ganglia as a latent infection. When the body's immunity declines, the virus can be reactivated and replicated massively, triggering an immune response in the peripheral sensory nerves and the unilateral dermatome innervated by such nerves, resulting in herpes zoster characterized by erythema, clustered varicella lesions and neuralgia.

Postherpetic neuralgia (PHN) is one of the most complex and common adverse complications among the clinical symptoms of herpes zoster, manifesting as burning pain or electric shock-like pain. Some patients also suffer from pruritic pain, mainly persistent pain and throbbing pain, and even hyperalgesia or allodynia, which severely affects patients' quality of life physically, psychologically, functionally and socially.

The main prevention and treatment methods for herpes zoster include active prevention with vaccines, antiviral drug therapy, and treatment of neuralgia caused by complications.

The incidence of herpes zoster increases with age and is gradually becoming younger. Complications such as neuralgia severely affect patients' lives, yet there is a lack of rapid and effective treatment methods. Vaccination can prevent herpes zoster. Given the above factors, the demand for herpes zoster vaccines is increasing year by year.

At present, only four herpes zoster vaccines have been approved for marketing globally, namely Zostavax from Merck & Co., SkyZoster from SK Chemicals, Ganwei from BCHT Biotechnology, and Shingrix from GlaxoSmithKline. SkyZoster is sold only in South Korea with a market share of approximately 1.0%; Ganwei was launched in 2023 and is sold only in China; Zostavax has been discontinued; Shingrix was approved by the FDA in 2017 and conditionally approved by the National Medical Products Administration in 2019, making it the first herpes zoster vaccine launched in China, and was officially marketed and sold in China in June 2020. Statistics show that Shingrix accounts for almost 100% of the market. Both Zostavax and Ganwei are live attenuated vaccines: the former is intended for people aged 50 and above with a protective efficacy of about 50% and requires one dose; the latter is intended for people aged 40 and above and also requires only one dose. Shingrix is a recombinant protein vaccine, in which gE protein is combined with AS01B adjuvant, intended for people aged 60 and above with a protective efficacy of over 90% and requiring two doses.

Current technical routes for the research and development of herpes zoster vaccines mainly include attenuated vaccines, recombinant protein vaccines, mRNA vaccines and adenovirus vaccines. In addition to the above-mentioned marketed attenuated vaccines, many domestic companies have entered clinical stages: for example, Wantai Biopharm's freeze-dried herpes zoster vaccine (VZV-7D) and the Shanghai Institute's live attenuated herpes zoster vaccine have both entered Phase II clinical trials; Changchun Qijian's live attenuated herpes zoster vaccine has obtained approval. Besides the marketed Shingrix, domestic recombinant vaccines are also under rapid progress: Luzhu Biopharmaceutical's recombinant vaccine adopts an innovative tetramer molecular structure and has entered Phase II clinical trials; Yidao Bio's recombinant herpes zoster vaccine has also entered Phase II clinical trials. mRNA vaccines are currently in the research and development stage with no marketed products yet. The chimpanzee adenovirus-vectored shingles vaccine co-developed by CanSino Biologics and Vaccitech (UK) has entered the preclinical stage.

### Summary of the Invention

To address technical defects in the prior art such as the limited variety of herpes zoster mRNA vaccine products and poor immune effects, the present invention provides a VZV antigen variant, a nucleic acid, a pharmaceutical composition and applications thereof. The VZV antigen variant can induce enhanced high immune response, for example, a stronger T-cell immune response, a potent cellular immune response, and a high level of cytokine production (such as TNF-α). The nucleic acid can achieve a high antigen expression level.

One technical solution provided by the present invention is as follows: the VZV antigen variant has a Y582G mutation compared with the amino acid sequence as shown in SEQ ID NO: 1;
and/or, the VZV antigen variant has a deletion of positions 561-623, 569-623 or 574-623 compared with the amino acid sequence as shown in SEQ ID NO: 1;
and/or, the VZV antigen variant is with modifications in the transmembrane and intracellular domains compared with the amino acid sequence as shown in SEQ ID NO: 1, wherein the modification of the protein transmembrane domain and intracellular domain consists in replacing the transmembrane domain and intracellular domain of a VZV antigen (e.g., a wild-type VZV antigen) with the transmembrane domain of SARS-CoV-2 Spike protein or the transmembrane domain of influenza H protein.

In the present invention, the VZV antigen variant is also referred to as a VZV antigen, except when the amino acid sequence is defined as the wild-type VZV antigen as shown in SEQ ID NO: 1.

One technical solution provided by the present invention is as follows: a VZV antigen, wherein the amino acid residue at position 582 of SEQ ID NO: 1 is substituted with G;
and/or, the VZV antigen has a deletion of positions 561-623, 569-623 or 574-623 based on SEQ ID NO: 1;
and/or, the VZV antigen is modified in the protein transmembrane domain and intracellular domain based on SEQ ID NO: 1, wherein the modification of the protein transmembrane domain and intracellular domain consists in replacing the transmembrane domain and intracellular domain of the wild-type VZV antigen with the transmembrane domain of SARS-CoV-2 Spike protein or the transmembrane domain of influenza H protein.

In some preferred embodiments, the VZV antigen variant further comprises one or more substitutions selected from Y569A, S593A, S595A, T596A and T598A compared with the amino acid sequence as shown in SEQ ID NO: 1; preferably, the VZV antigen variant further comprises Y569A, S593A, S595A, T596A and T598A compared with the amino acid sequence as shown in SEQ ID NO: 1;
and/or, the modification of the protein transmembrane domain and intracellular domain consists in replacing the transmembrane domain and intracellular domain of a VZV antigen (e.g., a wild-type VZV antigen) with the transmembrane domain of SARS-CoV-2 Spike protein; the transmembrane domain and intracellular domain of the VZV antigen correspond to positions 538-647 of SEQ ID NO: 1.

Preferably, the VZV antigen comprises one or more substitutions selected from substitution of amino acid residue at position 569 with A, substitution of amino acid residue at position 593 with A, substitution of amino acid residue at position 595 with A, substitution of amino acid residue at position 596 with A, and substitution of amino acid residue at position 598 with A based on SEQ ID NO: 1; preferably, the VZV antigen further comprises substitution of amino acid residue at position 569 with A, substitution of amino acid residue at position 593 with A, substitution of amino acid residue at position 595 with A, substitution of amino acid residue at position 596 with A, and substitution of amino acid residue at position 598 with A based on SEQ ID NO: 1;
and/or, the modification of the protein transmembrane domain and intracellular domain consists in replacing the transmembrane domain and intracellular domain of the wild-type VZV antigen with the transmembrane domain of SARS-CoV-2 Spike protein.

In a preferred embodiment of the present invention, the VZV antigen comprises a Hibit-HA-tag, the sequence of which is e.g., SEQ ID NO: 37.

In a preferred embodiment of the present invention, the transmembrane domain and intracellular domain of the VZV antigen correspond to positions 538-647 of SEQ ID NO: 1.

In a preferred embodiment of the present invention, the amino acid sequence of the transmembrane domain of SARS-CoV-2 Spike protein is as shown in SEQ ID NO: 10.

In a preferred embodiment of the present invention, the amino acid sequence of the transmembrane domain of influenza H protein is as shown in SEQ ID NO: 11.

In a preferred embodiment of the present invention, the amino acid sequence of the VZV antigen comprises an amino acid sequence as shown in any one of SEQ ID NO: 2-9.

In the present invention, uppercase letters represent amino acids in single-letter code, the meanings of which are well known to those skilled in the art. In the present invention, numbers represent positions in the amino acid sequence before deletion or substitution of an amino acid; a single uppercase letter before a number generally represents the amino acid at that position before deletion or substitution, and a single uppercase letter after a number generally represents the amino acid at that position after substitution. Those skilled in the art can readily deduce the position represented by the number in the VZV gE antigen amino acid sequence according to the VZV gE antigen amino acid sequence.

To solve the above technical problems, another technical solution provided by the present invention is as follows: an isolated nucleic acid comprising a nucleotide sequence encoding the VZV antigen as described in the present invention.

In a preferred embodiment of the present invention, the isolated nucleic acid is mRNA. Preferably, the mRNA comprises one or more of Cap1, 5'UTR and 3'UTR-polyA.

In a specific embodiment of the present invention, the nucleotide sequence encoding the VZV antigen comprises a nucleotide sequence as shown in any one of SEQ ID NO: 15-25 and 27-36.

To solve the above technical problems, another technical solution provided by the present invention is as follows: an expression element comprising a starting plasmid and the isolated nucleic acid as described in the present invention.

In a preferred embodiment of the present invention, the starting plasmid is pcDNA3.1.

To solve the above technical problems, another technical solution provided by the present invention is as follows: a method for preparing the isolated nucleic acid as described in the present invention, comprising in vitro transcription of the expression element as described in the present invention. The in vitro transcription is a conventional in vitro transcription method in the art.

To solve the above technical problems, another technical solution provided by the present invention is as follows: a pharmaceutical composition comprising the VZV antigen as described in the present invention, the isolated nucleic acid or the expression element as described in the present invention, and a lipid.

In a preferred embodiment of the present invention, the lipid is a composition composed of an ionizable lipid compound, DSPC (1,2-distearoyl-sn-glycero-3-phosphocholine), cholesterol and DMG-PEG2000 (1,2-dimyristoyl-rac-glycero-3-methoxypolyethylene glycol-2000). Preferably, the ratio of ionizable lipid compound, DSPC, cholesterol and DMG-PEG2000 is 50:10:38.5:1.5.

In a preferred embodiment of the present invention, the composition is prepared according to the following steps:
Firefly luciferase (Fluc) mRNA is diluted in 50 mM citrate buffer (pH 4.0) to obtain an mRNA solution. An ethanolic lipid solution and the mRNA solution are mixed at a flow rate of 12 mL/min and a volume ratio of 1:3 using a microfluidic device (e.g., NanoAssemblr (manufacturer: Precision Nanosystems) microfluidic mixing system) to prepare lipid nanoparticles at an N/P ratio of ionizable lipid to mRNA of 3-15:1. Ethanol is removed by dialysis against 0.01 M phosphate-buffered saline (PBS) for 12-24 h. Finally, the lipid nanoparticle solution is filtered through a 0.22 µm sterile filter and concentrated by ultrafiltration (Amicon-Ultra, MWCO 10 kDa) to obtain an LNP formulation encapsulating Fluc mRNA using ionizable lipid/DSPC/cholesterol/DMG-PEG2000.

In a preferred embodiment of the present invention, the pharmaceutical composition comprises an amino acid sequence as shown in SEQ ID NO: 5 or SEQ ID NO: 8, and the lipid comprises LQ104 or E16b2.

In a preferred embodiment of the present invention, the pharmaceutical composition is a vaccine formulation. Preferably, the vaccine formulation further comprises an adjuvant.

To solve the above technical problems, another technical solution provided by the present invention is as follows: use of the VZV antigen as described in the present invention, the isolated nucleic acid as described in the present invention, or the pharmaceutical composition as described in the present invention in the preparation of a medicament for preventing VZV infection. Preferably, the VZV infection is chickenpox or herpes zoster, such as postherpetic neuralgia.

On the basis of conforming to common general knowledge in the art, the above preferred conditions can be combined arbitrarily to obtain preferred examples of the present invention.

All reagents and raw materials used in the present invention are commercially available.

The positive and progressive effects of the present invention are as follows:
The VZV antigen provided by the present invention has stronger immunogenicity than the prior art VZV antigen, can achieve a high binding antibody titer, and has higher protective efficacy. The vaccine prepared from the VZV antigen nucleotide sequence of the present invention has a humoral immune response and cellular immune response non-inferior to the VZV vaccine of Shingrix (with some aspects showing superior efficacy), and has broad clinical application prospects.

### Brief Description of the Drawings

Figure 1 shows in vitro expression of varicella-zoster virus vaccine antigen.
Figure 2 shows in vitro expression of candidate antigen sequences after LNP encapsulation.
Figure 3 shows the immunization schedule of the preliminary screening antigens in C57BL/6J mice.
Figure 4 shows humoral immune responses induced in mice by candidate vaccines.
Figure 5 shows cellular immune responses induced in mice by candidate vaccines, in which panel A is a bar chart of IL-2, IFN-γ and TNF-α cytokine levels produced by CD4+ T cells and CD8+ T cells, and panel B is a stacked bar chart of IL-2, IFN-γ and TNF-α cytokine levels produced by CD4+ T cells and CD8+ T cells.
Figure 6 shows in vitro expression of optimized varicella-zoster virus vaccine antigens.
Figure 7 shows in vitro expression of optimized antigen sequences after LNP encapsulation.
Figure 8 shows the immunization procedure of optimized antigens in C57BL/6J mice.
Figure 9 shows humoral immune responses induced in mice by optimized candidate vaccines, in which panel A shows antibody levels induced in mice at a dose of 1 µg, and panel B shows antibody levels induced in mice at a dose of 5 µg.
Figure 10 shows cellular immune responses induced in mice by 1 µg optimized candidate vaccines; panel A shows IFN-γ levels in mice; panel B shows IL-2 levels in mice.
Figure 11 shows cellular immune responses induced in mice by 5 µg optimized candidate vaccines; panel A shows IFN-γ levels in mice; panel B shows IL-2 levels in mice.
Figure 12 shows the immunization schedule of optimized antigens in guinea pigs.
Figure 13 shows humoral immune responses induced in guinea pigs by optimized candidate vaccines, in which panel A shows antibody levels induced in guinea pigs at a dose of 5 µg, and panel B shows antibody levels induced in guinea pigs at a dose of 25 µg.
Figure 14 shows cellular immune responses induced in guinea pigs by optimized candidate vaccines.
Figure 15 shows the structure of mRNA; all mRNA structures shown contain the structural elements of "VZVE", and VZVE-1, 2, 3, 4, 5, 6 and 7 are not shown repeatedly.

### Detailed Description of the Invention

The present invention is further illustrated by the following examples, but is not thereby limited to the scope of the examples described herein. For experimental methods in the following examples without specified conditions, selection shall be made in accordance with conventional methods and conditions, or in accordance with product manuals.

### Example 1 RQ3400 - Screening of Herpes Zoster Vaccine Antigens

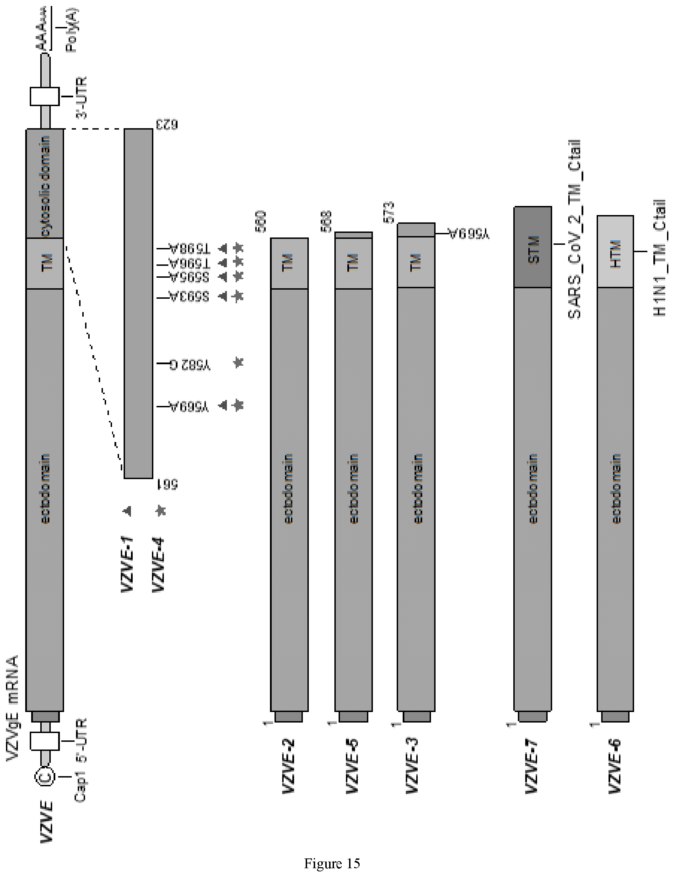

Different mutants or truncated forms of VZV gE protein were screened by in vitro antigen expression. The antigen sequences of VZVE, VZVE-1, VZVE-2, VZVE-3, VZVE-4 and VZVE-5 were separately synthesized by chemical synthesis. The antigen sequences were codon-optimized for human expression, and three independent syntheses of each were prepared as templates for in vitro transcription. UTP was replaced with pseudouridine-5'-triphosphate (Pseudo-UTP) in the mRNAs (structures shown in Figure 15). The mRNAs were transfected into 293T cells (Thermo Fisher, Cat. No.: K1538), and the expression of each antigen sequence was verified by Western blot analysis. The specific steps are as follows:

### I. In Vitro Transcription and mRNA Transfection

### 1. Obtaining Linearized Target Fragment by PCR

According to the requirements of the PCR reaction, add 8.7 µL ddH₂O, 0.5 µL template DNA (source: synthesized by GenScript), 0.4 µL forward and 0.4 µL reverse primers (source: synthesized by GenScript), and 10 µL PrimerSTAR MAX polymerase (source: TAKARA, Cat. No. R450A) into a 1.5 mL centrifuge tube, and mix well.

As shown in Table 1:

**Table 1 PCR System**

| | |
|---|---|
| Plasmid | 0.5 µL |
| Primer F: T7-mRNA-pCDNA-F | 0.4 µL |
| Primer R: Human-HBA-UTR-100T-R | 0.4 µL |
| H₂O | 8.7 µL |
| PrimerSTAR Max | 10 µL |
| Total | 20 µL |

The final PCR reaction conditions are shown in Table 2:

**Table 2 PCR Conditions**

| | | |
|---|---|---|
| 98°C | 3 min | |
| 98°C | 15 s | 34x |
| 60°C | 10 s | |
| 72°C | 80s (30 s/kb) | |
| 72°C | 10 min | |
| 10°C | ∞ | |

After the PCR reaction, perform gel electrophoresis verification. If the nucleic acid bands are uniform, proceed to the next IVT reaction; if not, repeat the PCR.

### 2. In Vitro Transcription (IVT)

A small-scale IVT test was performed according to the formulation in Table 3 (20 µL system):

**Table 3 IVT System**

| | |
|---|---|
| ddH₂O | 11 µL |
| 10x HYB (Source: ApexBio, Catalog No.: K1083) | 2 µL |
| Cap1 (B8176, 100 mM) (Source: ApexBio, Catalog No.: B8176) | 0.4 µL |
| GTP (100 mM) (Source: Hongene, Catalog No.: R2331) | 0.4 µL |
| ATP (100 mM) (Source: Hongene, Catalog No.: R1331) | 0.4 µL |
| CTP (100 mM) (Source: Hongene, Catalog No.: R3331) | 0.4 µL |
| Pseudo-U (100 mM) (Source: Hongene, Catalog No.: R5331) | 0.4 µL |
| T7 mix (Source: ApexBio, Catalog No.: K1083) | 2 µL |
| PCR product | 3 µL |
| Total | 20 µL |

After preparation, place the entire reaction system in a 37°C water bath and incubate for 2 h. After the reaction, digest the DNA template by adding 1 µL DNase I (source: Vazyme, Cat. No. EN401-01) (35 µL for 1 mL IVT reaction), incubate at 37°C for 15 min, then proceed with IVT recovery. The recovery steps are as follows:
I. Add 80 µL ddH₂O to the 20 µL digestion product to bring the volume to 100 µL.
II. Add 350 µL Solution D and 250 µL anhydrous ethanol (source: Hushi, Cat. No. 801769722), mix thoroughly, transfer to a nucleic acid purification column (source: Solarbio, Cat. No. N1012), and centrifuge at 10,000 g for 1 min.
III. Add 500 µL 70% ethanol, centrifuge at 10,000 g for 1 min, repeat once, then centrifuge at 10,000 g for 2 min.
IV. Add 70 µL sodium citrate (source: Sigma, Cat. No. C8532) to the purification column, let stand at room temperature for 1 min, then centrifuge at 10,000 g for 2 min. Collect the flow-through, which is the target RNA.

Prepare a 1% agarose gel according to the above formulation and perform RNA electrophoresis at 160 V for 20 min. After electrophoresis, observe under a gel imaging system (source: Tanon, Cat. No. Tanon 4600 SF). If the band is single, proceed with concentration determination.

### 3. Cell Transfection

Incubate 1 µg mRNA sample with 2 µL lipo2000 at room temperature for 20 min, then add the mixed sample to 1e6 293T cells and culture at 37°C, 5% CO₂ for 18-20 h, then collect the cells.

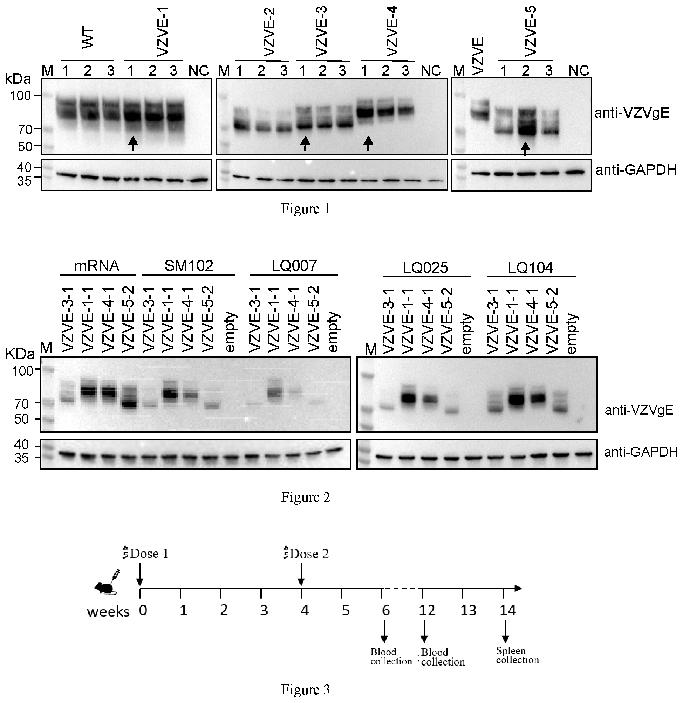

The in vitro expression results of the varicella-zoster virus vaccine antigen are shown in Figure 1. The arrow-marked sequences, namely VZVE-1-1, VZVE-3-1, VZVE-4-1, and VZVE-5-2, showed good expression in 293T cells. NC is the negative control, and no corresponding band was detected. These four antigen sequences were selected as candidate sequences for LNP encapsulation. In addition, the VZVE-4-3 mRNA sequence has a Y581G mutation based on VZVE (mRNA sequence as shown in SEQ ID NO:26), which can be used as a comparison. The amino acid sequence of VZVE-4-3 is shown in SEQ ID NO:38.

The above candidate antigen sequences VZVE-1-1, VZVE-3-1, VZVE-4-1, and VZVE-5-2 were used as templates for in vitro transcription, and UTP was replaced with Pseudo-UTP in the mRNA sequences. The lipids used for encapsulation were independently developed by RNACure, including LQ007, LQ025, and LQ104.

Preparation of lipid compound LQ007:

### Step 1: Preparation of LQ007-1

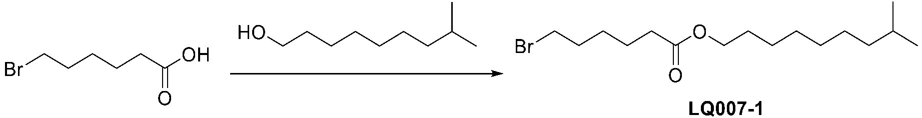

The material ratios are shown in Table 4:

**Table 4 Material Ratios**

| Material Name | Molecular Weight | Molar Ratio | Amount | mmol |
|---|---|---|---|---|
| 6-Bromohexanoic acid | 195 | 1.2eq | 2.34g | 12 |
| Isodecyl alcohol | 158 | 1eq | 1.58g | 10 |
| DCC | 206 | 2eq | 4.12g | 20 |
| DMAP | 122 | 0.1eq | 122mg | 1 |
| DCM | - | - | 50mL | - |

### Procedure:

Add 6-bromohexanoic acid, isodecyl alcohol, DCC, DMAP, and DCM to a reaction flask and stir at room temperature for 12 h. TLC (EA:PE=20:1) showed complete reaction.

### Work-up:

Filter the reaction mixture through diatomaceous earth, evaporate to dryness, and purify by column chromatography to obtain 2 g of colorless oil.

TLC: EA:PE=20:1, product Rf=0.6.

### Step 2: Preparation of LQ001-1

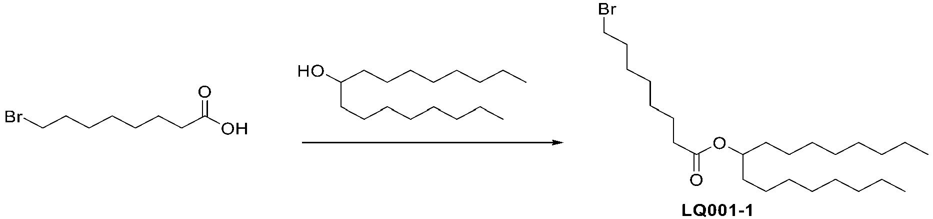

The material ratios are shown in Table 5:

**Table 5 Material Ratios**

| Material Name | Molecular Weight | Molar Ratio | Amount | mmol |
|---|---|---|---|---|
| 8-Bromooctanoic acid | 223 | 1.1eq | 24.5g | 110 |
| 9-Heptadecanol | 256 | 1eq | 25.6g | 100 |
| DCC | 206 | 2eq | 41.2g | 200 |
| DMAP | 122 | 0.1eq | 1.22g | 10 |
| DCM | - | - | 500mL | - |

### Procedure:

Add 8-bromooctanoic acid, 9-heptadecanol, DCC, DMAP, and DCM to a reaction flask and stir at room temperature for 12 h. TLC (EA:PE=20:1) showed complete reaction.

### Work-up:

Filter the reaction mixture through diatomaceous earth, dry, and purify by column chromatography to obtain 40 g of colorless oil, a yield of 85%.

¹ H NMR (500 MHz, CDCl₃) δ: 4.87 (p, 1H), 3.40 (t, 2H), 2.28 (t, 2H), 1.85 (p, 2H), 1.63 (p, 2H), 1.54-1.47 (m, 4H), 1.43 (dt, 2H), 1.33 (dt, 4H), 1.27 (d, 24H), 0.88 (t, 6H).
:

### Step 3: Preparation of LQ001-2

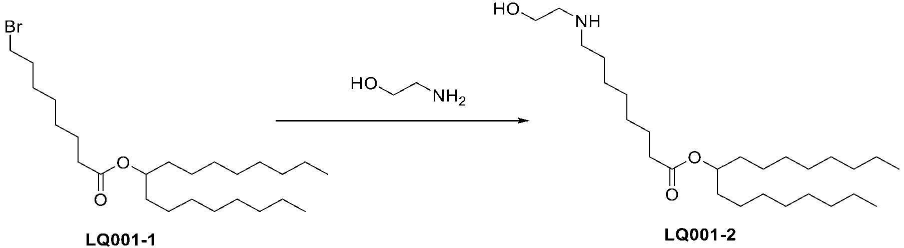

The material ratios are shown in Table 6:

**Table 6 Material Ratios**

| Material Name | Molecular Weight | Molar Ratio | Amount | mmol |
|---|---|---|---|---|
| LQ001-1 | 460 | 1eq | 30g | 65.2 |
| Ethanolamine | 61 | 10eq | 40g | 652 |
| Acetonitrile | - | - | 400mL | - |

### Procedure:

Add LQ001-1, ethanolamine, and acetonitrile to a reaction flask, heat to 30°C and stir for 12 h. TLC (EA:PE=20:1) showed complete reaction.

### Work-up:

Evaporate the reaction mixture to dryness, dilute with 500 mL ethyl acetate, wash twice with 500 mL water, dry the organic phase over anhydrous sodium sulfate, evaporate to dryness, and purify by column chromatography to obtain 25 g of colorless oil, a yield of 87%.

¹ H NMR (500 MHz, CDCl₃) δ: 4.86 (p, 1H), 3.65-3.61 (m, 2H), 2.80-2.75 (m, 2H), 2.61 (t, 2H), 2.27 (t, 2H), 1.62 (p, 3H), 1.48 (dt, 7H), 1.32 (s, 7H), 1.27 (d, 22H), 0.87 (t, 6H).

### Step 4: Preparation of LQ007

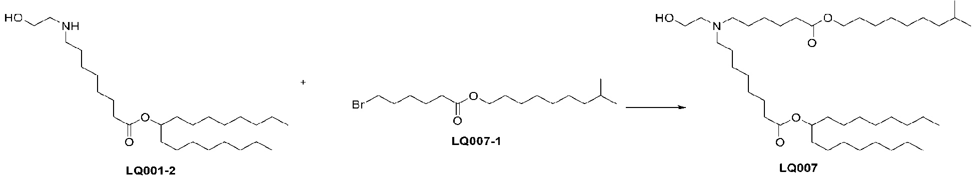

The material ratios are shown in Table 7:

**Table 7 Material Ratios**

| Material Name | Molecular Weight | Molar Ratio | Amount | mmol |
|---|---|---|---|---|
| LQ001-2 | 441 | 1eq | 1.6g | 3.75 |
| LQ007-1 | 335 | 1.2eq | 1.35g | 4.5 |
| K₂CO₃ | 138 | 3.5eq | 1.8g | 13 |
| KI | 166 | 1.2eq | 750mg | 4.5 |
| Acetonitrile | - | - | 20mL | - |
| Methyl tert-butyl ether | - | - | 20mL | - |

### Procedure:

Add LQ001-2, LQ007-1, K₂CO₃, KI, methyl tert-butyl ether, and acetonitrile to a reaction flask, heat to 80°C and stir for 12 h. TLC (DCM:MeOH=10:1) showed complete reaction.

### Work-up:

Filter the reaction mixture, dry, and purify by column chromatography to obtain 2 g of colorless oil, a yield of 80%.

¹ H NMR (500 MHz, CDCl₃) δ: 4.86 (p, 1H), 4.14-4.02 (m, 2H), 3.56 (t, 2H), 2.61 (t, 2H), 2.48 (q, 4H), 2.32-2.25 (m, 4H), 1.68-1.58 (m, 6H), 1.56-1.40 (m, 10H), 1.36-1.21 (m, 38H), 1.19-1.03 (m, 4H), 0.87 (t, 12H).

LCMS: Rt: 3.83 min; MS-m/z (ESI): 696.7 [M+H]⁺.

### Preparation of lipid compound LQ025:

### Step 1: Preparation of LQ025-1

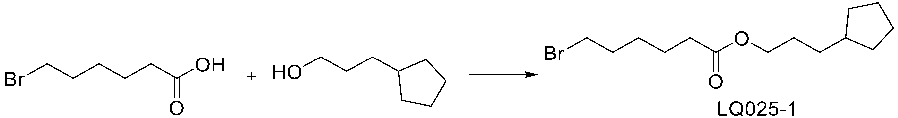

The material ratios are shown in Table 8:

**Table 8 Material Ratios**

| Material Name | Molecular Weight | Molar Ratio | Amount | mmol |
|---|---|---|---|---|
| 6-Bromohexanoic acid | 195 | 1.2eq | 2.34g | 12 |
| 3-Cyclopentyl-1-propanol | 128 | 1eq | 1.28g | 10 |
| DCC | 206 | 2eq | 4.12g | 20 |
| DMAP | 122 | 0.1eq | 122mg | 1 |
| DCM | - | - | 50mL | - |

### Procedure:

Add 6-bromohexanoic acid, 3-cyclopentyl-1-propanol, DCC, DMAP, and DCM to a reaction flask and stir at room temperature for 12 h. TLC (EA:PE=20:1) showed complete reaction.

### Work-up:

Filter the reaction mixture through diatomaceous earth, evaporate to dryness, and purify by column chromatography to obtain 2.8 g of colorless oil.

TLC: EA:PE=20:1, product Rf=0.6.

### Step 2: Preparation of LQ025

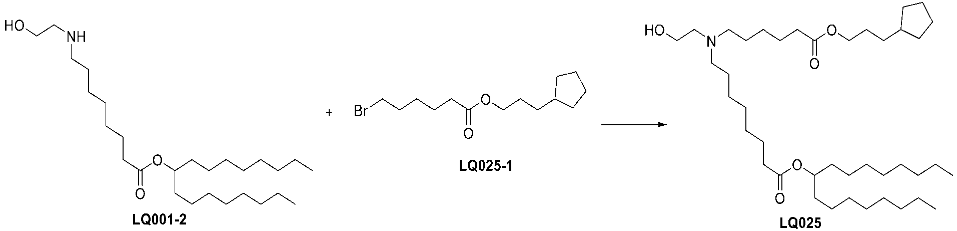

The material ratios are shown in Table 9:

**Table 9 Material Ratios**

| Material Name | Molecular Weight | Molar Ratio | Amount | mmol |
|---|---|---|---|---|
| LQ001-2 | 441 | 1eq | 1.6g | 3.75 |
| LQ025-1 | 305 | 1.2eq | 1.35g | 4.5 |
| K₂CO₃ | 138 | 3.5eq | 1.8g | 13 |
| KI | 166 | 1.2eq | 750mg | 4.5 |
| Acetonitrile | - | - | 20mL | - |
| Methyl tert-butyl ether | - | - | 20mL | - |

### Procedure:

Add LQ001-2, LQ025-1, K₂CO₃, KI, methyl tert-butyl ether, and acetonitrile to a reaction flask, heat to 80°C and stir for 12 h. TLC (DCM:MeOH=10:1) showed complete reaction, product Rf=0.4.

### Work-up:

Filter the reaction mixture, dry, and purify by column chromatography to obtain 2 g of colorless oil, a yield of 80%.

¹ H NMR (500 MHz, CDCl₃) δ: 4.86 (p, 1H), 4.05 (t, 2H), 3.58 (t, 2H), 2.64 (t, 2H), 2.55-2.46 (m, 4H), 2.29 (dt, 4H), 1.75 (td, 3H), 1.69-1.56 (m, 8H), 1.55-1.43 (m, 10H), 1.37-1.28 (m, 10H), 1.25 (s, 23H), 1.06 (d, 3H), 0.87 (t, 6H).

LCMS: Rt: 3.83 min; MS m/z (ESI): 666.7 [M+H]⁺.

### Preparation of lipid compound LQ104:

### Step 1: Preparation of LQ001-1

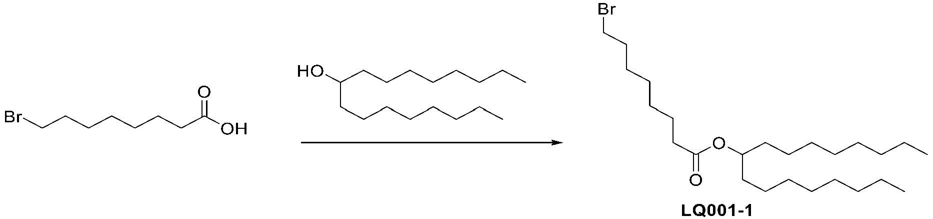

The material ratios are shown in Table 10:

**Table 10 Material Ratios**

| Material Name | Molecular Weight | Molar Ratio | Amount | mmol |
|---|---|---|---|---|
| 8-Bromooctanoic acid | 223 | 1.1eq | 24.5g | 110 |
| 9-Heptadecanol | 256 | 1eq | 25.6g | 100 |
| DCC | 206 | 2eq | 41.2g | 200 |
| DMAP | 122 | 0.1eq | 1.22g | 10 |
| DCM | - | - | 500mL | - |

### Procedure:

Add 8-bromooctanoic acid, 9-heptadecanol, DCC, DMAP, and DCM to a reaction flask and stir at room temperature for 12 h. TLC (EA:PE=20:1) showed complete reaction.

### Work-up:

Filter the reaction mixture through diatomaceous earth, dry, and purify by column chromatography to obtain 40 g of colorless oil, a yield of 85%.

¹ H NMR (500 MHz, CDCl₃) δ: 4.87 (p, 1H), 3.40 (t, 2H), 2.28 (t, 2H), 1.85 (p, 2H), 1.63 (p, 2H), 1.54-1.47 (m, 4H), 1.43 (dt, 2H), 1.33 (dt, 4H), 1.27 (d, 24H), 0.88 (t, 6H).

### Step 2: Preparation of LQ104

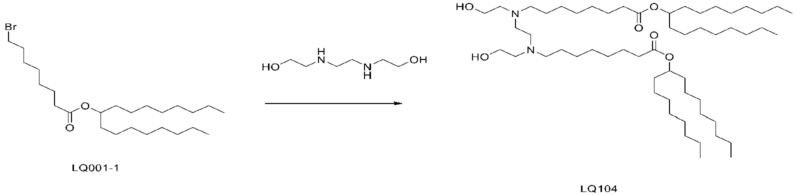

The material ratios are shown in Table 11:

**Table 11 Material Ratios**

| Material Name | Molecular Weight | Molar Ratio | Amount | mmol |
|---|---|---|---|---|
| LQ001-1 | 461.5 | 2.6eq | 4g | 8.5 |
| N,N'-Bis(2-hydroxyethyl)ethylenediamine | 148 | 1eq | 487mg | 3.3 |
| K₂CO₃ | 138 | 3.5eq | 1.6g | 11.5 |
| KI | 166 | 1.2eq | 655mg | 4.0 |
| Acetonitrile | - | - | 15ml | - |
| Methyl tert-butyl ether | - | - | 15ml | - |

### Procedure:

Add LQ001-1, N,N'-bis(2-hydroxyethyl)ethylenediamine, K₂CO₃, KI, methyl tert-butyl ether, and acetonitrile to a reaction flask, heat to 80°C and stir for 12 h. TLC (DCM:MeOH=10:1) showed complete reaction.

### Work-up:

Filter the reaction mixture, evaporate to dryness, and purify by column chromatography to obtain 1.1 g of colorless oil.

¹ H NMR (400 MHz, CDCl₃) δ: 4.86 (p, 2H), 3.65-3.59 (m, 4H), 2.68-2.59 (m, 8H), 2.57-2.49 (m, 4H), 2.27 (t, 4H), 1.61 (p, 5H), 1.49 (dt, 12H), 1.28 (d, 61H), 0.87 (t, 12H).

### Preparation of lipid compound E16b2:

### Step 1: Preparation of Compound 1

### Reaction:

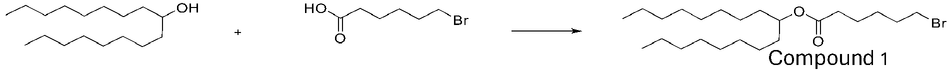

The material ratios are shown in Table 12:

**Table 12 Material Ratios**

| Material Name | Molecular Weight | Molar Ratio | Amount | mmol |
|---|---|---|---|---|
| 9-Heptadecanol | 256.47 | 1eq | 12.8g | 50 |
| 6-Bromohexanoic acid | 195.06 | 1.1eq | 10.7g | 55 |
| DCC | 206 | 2eq | 20.6g | 100 |
| DMAP | 122 | 0.1eq | 610mg | 5 |
| DCM | | | 300ml | |

### Procedure:

Add 6-bromohexanoic acid, DCC, DMAP, and DCM to a 1 L reaction flask, then add 9-heptadecanol, and stir at room temperature for 12 h. TLC (PE:EA=20: 1) showed complete reaction (product Rf = 0.6).

### Work-up:

Filter the reaction mixture, evaporate to dryness, and purify by column chromatography to obtain 14.4 g of colorless oil.

### Step 2: Preparation of E16b2

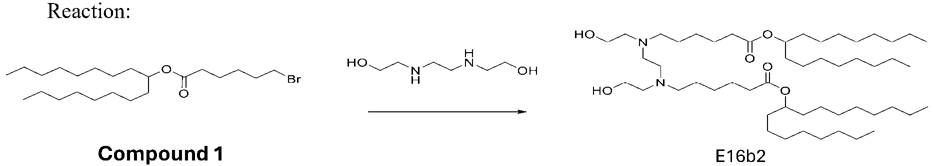

The material ratios are shown in Table 13:

**Table 13 Material Ratios**

| Material Name | Molecular Weight | Molar Ratio | Amount | mmol |
|---|---|---|---|---|
| Compound 1 | 433.5 | 2.5eq | 2.17g | 5 |
| N,N'-Bis(2-hydroxyethyl)ethylenediamine | 148 | 1eq | 300mg | 2 |
| K₂CO₃ | 138 | 4eq | 1.1g | 8 |
| KI | 166 | 2eq | 664mg | 4 |
| Acetonitrile | | | 40ml | |

### Procedure:

Add compound 1, N,N'-bis(2-hydroxyethyl)ethylenediamine, K₂CO₃, KI, and acetonitrile to a reaction flask, heat to 80°C and stir for 12 h. TLC (DCM:MeOH=10:1) showed complete reaction (product Rf = 0.5).

### Work-up:

Filter the reaction mixture, rotary evaporate to dryness, and purify by column chromatography to obtain 770 mg of colorless oil.

¹ H NMR (600 MHz, CDCl₃) δ: 4.85 (p, J=6.2 Hz, 2H), 3.65 (t, J=4.8 Hz, 4H), 2.73-2.65 (m, 8H), 2.61 (t, J=8.1 Hz, 4H), 2.28 (t, J=7.4 Hz, 4H), 1.64 (p, J=7.5 Hz, 4H), 1.51 (dq, J=19.0, 7.3, 6.8 Hz, 12H), 1.35-1.20 (m, 54H), 0.87 (t, J=6.9 Hz, 12H).

Using SM102 lipid as a control, the preparation method for the lipids used is as follows:
Dissolve the series of ionizable lipid compounds, 1,2-distearoyl-sn-glycero-3-phosphocholine (DSPC, Nippon Fine Chemical Co., Ltd., Cat. No. S01005), cholesterol (Nippon Fine Chemical Co., Ltd., Cat. No. O01001), and 1,2-dimyristoyl-rac-glycero-3-methoxypolyethylene glycol-2000 (DMG-PEG2000, Guobang Pharma, Cat. No. O02005) in ethanol solution. Adjust the ratio of the four lipid components (ionizable lipid compound : cholesterol : DSPC : DMG-PEG2000 = 50 : 38.5 : 10 : 1.5) to prepare a mixed lipid ethanol solution (total lipid concentration of 12.5 mM).Dilute the above four candidate antigen mRNAs in 50 mM citrate buffer (pH 4.0) to obtain mRNA solutions. Prepare lipid nanoparticles by mixing the lipid ethanol solution and the mRNA solution using a NanoAssembler (manufacturer: Precision Nanosystems) microfluidic mixing system at a flow rate of 12 mL/min and a volume ratio of 1:3, with an ionizable lipid to mRNA nitrogen-to-phosphate ratio of 3-15:1. Dialyze against 0.01 M phosphate-buffered saline (PBS) for 12-24 h to remove ethanol. Finally, filter the lipid nanoparticle solution through a 0.22 µm sterile filter and concentrate by ultrafiltration (Amicon-Ultra, MWCO 10 KDa) to obtain LNP formulations encapsulating the candidate antigen mRNAs using ionizable lipid/DSPC/cholesterol/DMG-PEG2000.

Transfect the packaged LNP-mRNA (i.e., lipid-encapsulated mRNA), LNP-empty (empty lipid nanoparticles without mRNA), and the corresponding mRNAs into 293T cells, and verify their in vitro expression by Western blot (Anti-VZVgE: sc-56995, Santa Cruz; Anti-GAPDH: 60004-1-1g, Proteintech).As shown in Figure 2, both mRNAs (positive control) and LNP-mRNAs were expressed. The LQ104 lipid-encapsulated formulation showed the best expression. The SM102 lipid-encapsulated formulation showed lower expression than LQ104. SM102 and LQ025 lipid-encapsulated formulations showed similar expression levels. LQ007 lipid-encapsulated formulation showed the weakest expression. LNP-empty served as a negative control, and no corresponding band was detected.

### Example 2: Evaluation of Immunogenicity of Candidate Vaccines in C57BL/6J Mice

In this example, 6-8 week old female C57BL/6J mice (Shanghai Lingchang Biotechnology Co., Ltd., SPF (Suzhou) Biotechnology Co., Ltd.) were selected, with 8 or 16 animals per group. Mice in each group were administered on week 0 and week 4, including placebo (PBS, P1010-100*2L, Solarbio) and encapsulated candidate vaccines. Intramuscular injection was used at a dose of 1 µg/mouse (i.e., each mouse was injected with a vaccine formulation containing 1 µg of mRNA). Blood was collected and serum was separated at week 2 and week 8 after the second administration for later use.At week 14 after the second administration, mouse spleens were collected (experimental strategy shown in Figure 3), and mouse splenocytes were isolated for later use.

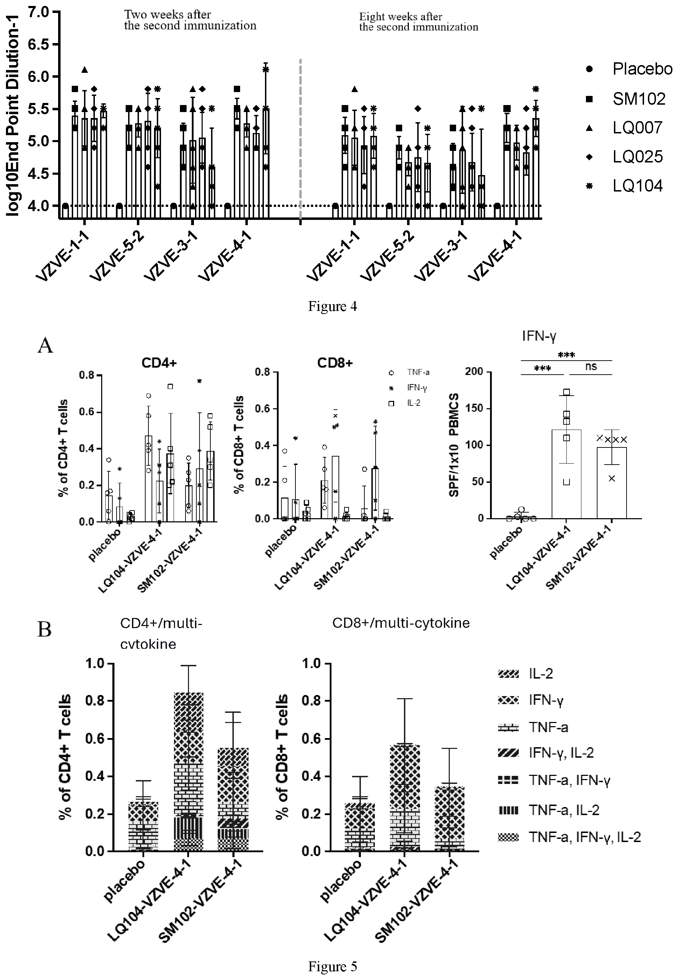

The antigen (VZV gE)-specific antibody titers in serum at week 2 and week 8 after the second administration were detected by indirect ELISA (coating antigen: Varicella-zoster virus (strain Oka vaccine) Envelope Glycoprotein E (gE), His Tag VZV gE, Cat. No.: Acrobiosystems, GLE-V52H3; secondary antibody: Goat Anti-mouse IgG(H+L), Cat. No.: C030205, BAI AOTONG; chromogenic solution: 1×TMB substrate solution (APEXBio, Cat#: K1131), Cat. No.: 002023 (Thermo Fisher); ELISA 96-well plate: 514201, NEST). The results are shown in Figure 4. At week 2 and week 8, the encapsulated candidate vaccines induced VZV gE-specific antibodies in mice, among which LQ104-VZVE-4-1 showed the best effect.

Based on the results of antigen-specific antibody levels induced by the candidate vaccines in mice, spleen samples from the LQ104-VZVE-4-1 and SM102-VZVE-4-1 groups were collected at week 14 after the second administration. The levels of IL-2, IFN-γ, and TNF-α cytokines produced by CD4+ T cells and CD8+ T cells were detected by flow cytometry and ELISpot methods.

### 1. Antigen-Specific T Cell Response Assay

Antigen-specific T cell responses in mouse spleens were measured by intracellular cytokine staining (ICS). Briefly, gE peptide library (covering the full-length gE protein, each peptide 15 mers with 11 amino acid overlap between peptides, synthesized by: GenScript) or medium containing an equal amount of DMSO was added to 96-well plates as a negative control. Mouse splenocytes resuspended in RPMI 1640 complete medium (Cat. No.: 6016011, purchased from Dyou) were added, incubated at 37°C for 1 hour, then protein transport inhibitor (Cat. No.: 554724, purchased from BD Bioscience) was added and incubated for an additional 5 hours. Cells were washed once with PBS and stained with Fixable-Viability-Stain-510 (Cat. No.: 564406, purchased from BD Bioscience). After incubation for 10 minutes, cells were washed and then anti-mouse CD16/CD32 (Cat. No.: 553142, purchased from BD Bioscience) was added and incubated at 4°C for 10 minutes. A mixture of anti-surface marker antibodies: anti-mouse CD3-FITC, CD4-APC, and CD8-Percp-cy5.5 (Cat. Nos.: 553061, 553051, 551162 respectively, purchased from BD Bioscience) was added for staining. After incubation for 30 minutes, cells were washed twice, fixed and permeabilized for 20 minutes, washed once, and stained with a mixture of anti-cytokine antibodies: anti-mouse IFN-γ-Pe-Cy7, IL-2-BV605, TNF-α-BV650, IL-4-BV711, and IL-5-PE (Cat. Nos.: 557649, 563943, 563911, 564005, and 562049 respectively, all purchased from BD Bioscience). After incubation for 30 minutes, cells were washed twice and resuspended in 200 µL PBS. Fluorescence signals were analyzed using a CYTEK-Aurora/NL flow cytometer (Model: NL-CLC-V16B14R8, purchased from Cytek Biosciences).

### 2. ELISpot

Antigen-specific IFN-γ and IL-2 responses in mouse spleens were measured by enzyme-linked immunospot (ELISpot). Briefly, isolated mouse splenocytes were added to plates, and gE peptide library (same as ICS) or medium containing an equal amount of DMSO was added as a negative control. After 22 hours of culture, subsequent operations were performed according to the instructions of the mouse IFN-γ/ELISpot kit (Cat. No.: 2210006, purchased from Dyou) and the mouse IL-2/ELISpot kit (Cat. No.: 3441-4APW-10, purchased from MabTech). Spots were counted using an ELISpot spot counter (Model: AT-Spot-2100, purchased from SINSAGE).

As shown in parts A and B of Figure 5, compared with SM102-VZVE-4-1, LQ104-VZVE-4-1 induced a stronger T cell immune response.

### Example 3: Antigen Sequence Iteration of the Screened VZVE-4 Antigen

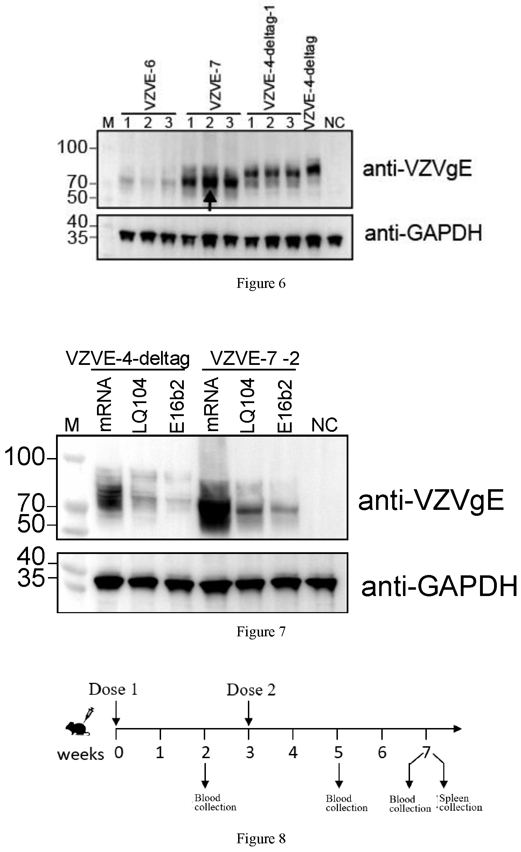

In this example, after preliminary screening of the VZVE-4 antigen, the Hibit-HA-tag (YPYDVPDYAGSSGVSGWRLFKKIS, SEQ ID NO:37) fused during screening was deleted from the VZVE-4 antigen to obtain the VZVE-4-deltag antigen sequence. In addition, three sequences (VZVE-4-deltag-1-1, VZVE-4-deltag-1-2, VZVE-4-deltag-1-3) were synthesized by codon optimization, using Pseudo-UTP. Subsequently, the transmembrane domain and intracellular domain of the original VZV gE protein were replaced with the transmembrane domain of the SARS-CoV-2 Spike protein (VZVE-7) or the transmembrane domain of the influenza H protein (VZVE-6). After codon humanization, three sequences were synthesized as templates for in vitro transcription, also using Pseudo-UTP. The mRNAs of VZVE-4-deltag, VZVE-4-deltag-1, VZVE-6, and VZVE-7 antigens were transfected into 293T cells, and their protein expression levels were detected by Western blot. As shown in Figure 6, VZVE-7-2 showed the highest expression level (i.e., among the codon-optimized sequences of VZVE-7, the second sequence had the best expression), followed by VZVE-4-deltag, then VZVE-4-deltag-1, while VZVE-6 showed poor expression levels.

Based on the Western blot results, the VZVE-7-2 and VZVE-4-deltag antigen sequences were selected and encapsulated using the new delivery lipids E16b2 and LQ104. The results are shown in Figure 7. LNP-mRNAs were expressed in 293T cells, and the antigen expression level of LQ104-mRNAs was slightly higher than that of E16b2-mRNAs.

### Example 4: Evaluation of Immunogenicity of Iterated Shingles Vaccine Antigens in C57BL/6J Mice

In this example, 6-8 week old female C57BL/6J mice were selected and divided into a placebo group, a positive control group (GSK marketed recombinant vaccine Shingrix, Cat. No.: HT4D4), and 4 iterated candidate vaccine groups (experimental groups). Each group contained 8 or 16 animals. Mice in each group were administered on week 0 and week 3 by intramuscular injection at doses of 1 µg/mouse and 5 µg/mouse for different groups, respectively. Blood was collected and serum was separated at week 2 after the first administration, and at week 2 and week 4 after the second administration for later use. At week 4 after the second administration, mouse spleens were collected and mouse splenocytes were isolated for later use (experimental strategy shown in Figure 8).

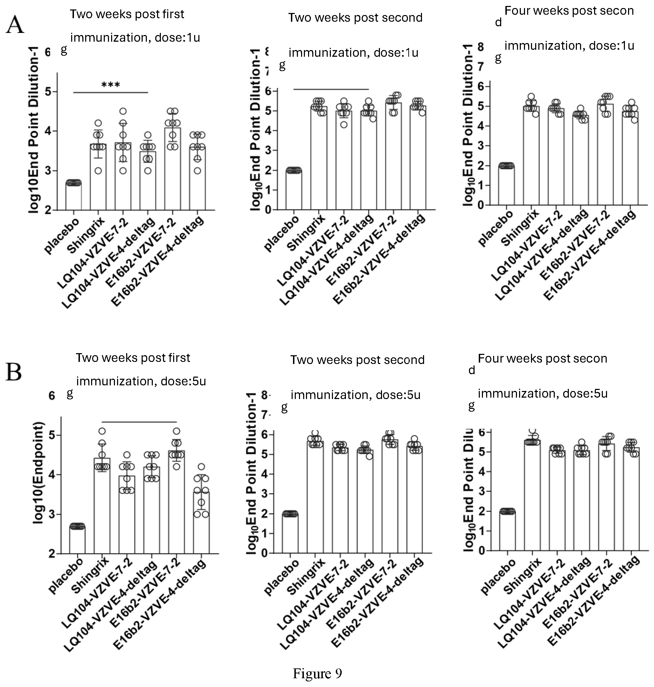

The antigen (VZV gE)-specific antibody titers in serum at week 2 after the first administration, and at week 2 and week 4 after the second administration were detected by indirect ELISA (using the same reagents as in Example 2). The results are shown in Figure 9. The iterated candidate vaccines induced VZV gE-specific antibodies in mice. At the 1 µg dose, the antibody level induced by E16b2-VZVE-7-2 was 1.5 to 2.5 times that of Shingrix (Figure 9, part A). At the 5 µg dose, the antibody level induced by E16b2-VZVE-7-2 was comparable to that of Shingrix (Figure 9, part B).

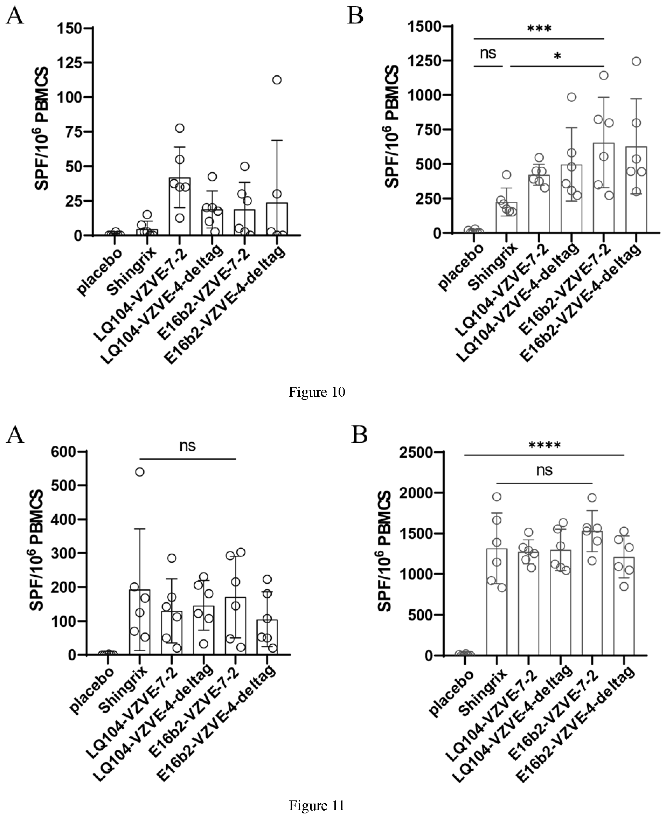

At week 4 after the second administration, mouse spleens were collected, and the levels of IL-2 and IFN-γ cytokines produced by antigen-induced CD4+ T cells were detected by flow cytometry and ELISpot (experimental methods and procedures same as in Example 2). Figure 10 shows that under the 1 µg dose immunization condition, the CD4+ T cell immune response elicited by the iterated candidate vaccines was non-inferior to that of Shingrix. For the IFN-γ cytokine, LQ104-VZVE-7-2 showed the highest level (Figure 10, part A), while for the IL-2 cytokine, E16b2-VZVE-7-2 showed the highest level and was significantly increased compared to Shingrix, indicating that E16b2-VZVE-7-2 induced a stronger cellular immune response (Figure 10, part B). As shown in parts A and B of Figure 11, under the 5 µg dose immunization condition, the CD4+ T cell immune response elicited by the iterated candidate vaccines was also non-inferior to that of Shingrix, and considering the levels of both cytokines, regardless of which lipid was used for encapsulation, VZVE-7-2 induced a stronger cellular immune response compared to VZVE-4-deltag.

### Example 5: Evaluation of Immunogenicity of Iterated Shingles Vaccine Antigens in Guinea Pigs

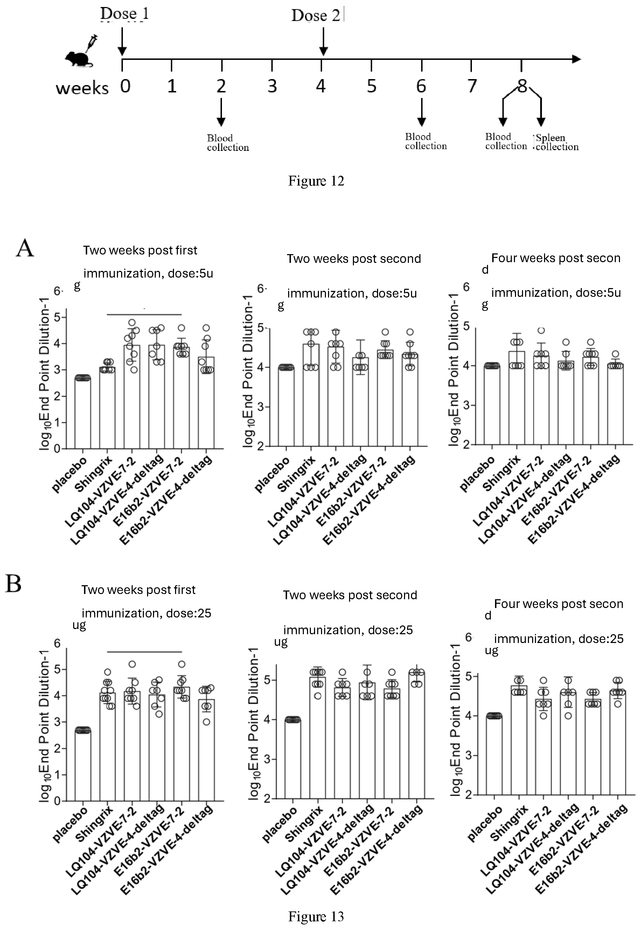

In this example, the immunogenicity of iterated shingles vaccine antigens was further evaluated in guinea pigs (Hartley strain guinea pigs, Jiashan Jintu Rabbit Industry Professional Cooperative). 6-8 week old female guinea pigs were selected and divided into a placebo group, a positive control group (GSK marketed recombinant vaccine Shingrix), and iterated candidate vaccine groups (4 experimental groups). Each group contained 8 or 16 animals. Guinea pigs in each group were administered on week 0 and week 4 by intramuscular injection at doses of 5 µg/guinea pig and 25 µg/guinea pig for both administrations. Blood was collected and serum was separated at week 2 after the first administration, and at week 2 and week 4 after the second administration for later use. At week 4 after the second administration, guinea pig spleens were collected and guinea pig splenocytes were isolated for later use (experimental strategy shown in Figure 12).

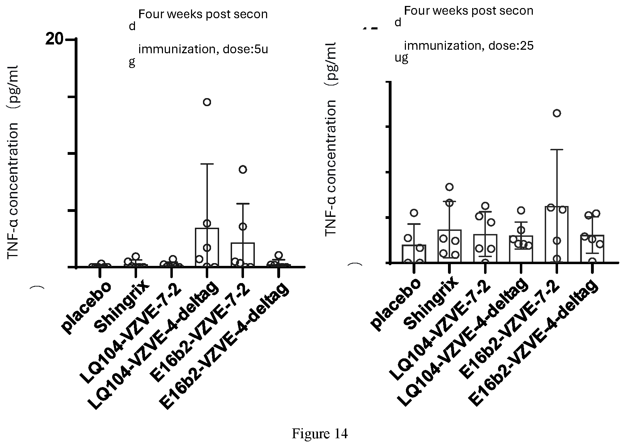

The antigen (VZV gE)-specific antibody titers in serum at week 2 after the first administration, and at week 2 and week 4 after the second administration were detected by indirect ELISA (using the same reagents as in Example 2), as shown in Figure 13. Figure 13 shows that the iterated candidate vaccines induced VZV gE-specific antibodies in guinea pigs, and at both the 5 µg (Figure 13, part A) and 25 µg (Figure 13, part B) doses, the antibody levels induced by E16b2-VZVE-7-2 were comparable to those of Shingrix. Figure 14 shows that at week 4 after the second administration, guinea pig spleens were collected, and antigen-induced T cell immune responses were detected by ELISA. Under the 5 µg dose immunization condition, the candidate vaccine LQ104-VZVE-4-deltag induced the highest level of TNF-α, followed by E16b2-VZVE-7-2, and both candidate vaccines induced higher TNF-α levels than Shingrix. Under the 25 µg dose immunization condition, the candidate vaccine E16b2-VZVE-7-2 induced the highest level of TNF-α.

The sequence information related to this application is shown in Table 14 below.

**Table 14 Antigen Amino Acid Sequences and mRNA Sequences**

| SEQ ID NO | Name | Sequence |
|---|---|---|
| 1 | VZVE amino acid sequence | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQ PTQMSAQEDLGDDTGIHVIPTLNGDDRHKIVNVDQRQYGDVFKGDLNPKPQGQRLIEV SVEENHPFTLRAPIQRIYGVRYTETWSFLPSLTCTGDAAPAIQHICLKHTTCFQDVVVDV DCAENTKEDQLAEISYRFQGKKEADQPWIVVNTSTLFDELELDPPEIEPGVLKVLRTEK QYLGVYIWNMRGSDGTSTYATFLVTWKGDEKTRNPTPAVTPQPRGAEFHMWNYHSH VFSVGDTFSLAMHLQYKIHEAPFDLLLEWLYVPIDPTCQPMRLYSTCLYHPNAPQCLSH MNSGCTFTSPHLAQRVASTVYQNCEHADNYTAYCLGISHMEPSFGLILHDGGTTLKFV DTPESLSGLYVFVVYFNGHVEAVAYTVVSTVDHFVNAIEERGFPPTAGQPPATTKPKEI TPVNPGTSPLLRYAAWTGGLAAVVLLCLVIFLICTAKRMRVKAYRVDKSPYNQSMYY AGLPVDDFEDSESTDTEEEFGNAIGGSHGGSSYTVYIDKTRYPYDVPDYAGSSGVSGWR LFKKIS |
| 2 | VZVE-1 amino acid sequence | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQ PTQMSAQEDLGDDTGIHVIPTLNGDDRHKIVNVDQRQYGDVFKGDLNPKPQGQRLIEV SVEENHPFTLRAPIQRIYGVRYTETWSFLPSLTCTGDAAPAIQHICLKHTTCFQDVVVDV DCAENTKEDQLAEISYRFQGKKEADQPWIVVNTSTLFDELELDPPEIEPGVLKVLRTEK QYLGVYIWNMRGSDGTSTYATFLVTWKGDEKTRNPTPAVTPQPRGAEFHMWNYHSH VFSVGDTFSLAMHLQYKIHEAPFDLLLEWLYVPIDPTCQPMRLYSTCLYHPNAPQCLSH MNSGCTFTSPHLAQRVASTVYQNCEHADNYTAYCLGISHMEPSFGLILHDGGTTLKFV DTPESLSGLYVFVVYFNGHVEAVAYTVVSTVDHFVNAIEERGFPPTAGQPPATTKPKEI TPVNPGTSPLLRYAAWTGGLAAVVLLCLVIFLICTAKRMRVKAARVDKSPYNQSMYY AGLPVDDFEDAEAADAEEEFGNAIGGSHGGSSYTVYIDKTRYPYDVPDYAGSSGVSGW RLFKKIS |
| 3 | VZVE-2 amino acid sequence | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQ PTQMSAQEDLGDDTGIHVIPTLNGDDRHKIVNVDQRQYGDVFKGDLNPKPQGQRLIEV SVEENHPFTLRAPIQRIYGVRYTETWSFLPSLTCTGDAAPAIQHICLKHTTCFQDVVVDV DCAENTKEDQLAEISYRFQGKKEADQPWIVVNTSTLFDELELDPPEIEPGVLKVLRTEK QYLGVYIWNMRGSDGTSTYATFLVTWKGDEKTRNPTPAVTPQPRGAEFHMWNYHSH VFSVGDTFSLAMHLQYKIHEAPFDLLLEWLYVPIDPTCQPMRLYSTCLYHPNAPQCLSH MNSGCTFTSPHLAQRVASTVYQNCEHADNYTAYCLGISHMEPSFGLILHDGGTTLKFV DTPESLSGLYVFVVYFNGHVEAVAYTVVSTVDHFVNAIEERGFPPTAGQPPATTKPKEI TPVNPGTSPLLRYAAWTGGLAAVVLLCLVIFLICTYPYDVPDYAGSSGVSGWRLFKKIS |
| 4 | VZVE-3 amino acid sequence | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQ PTQMSAQEDLGDDTGIHVIPTLNGDDRHKIVNVDQRQYGDVFKGDLNPKPQGQRLIEV SVEENHPFTLRAPIQRIYGVRYTETWSFLPSLTCTGDAAPAIQHICLKHTTCFQDVVVDV DCAENTKEDQLAEISYRFQGKKEADQPWIVVNTSTLFDELELDPPEIEPGVLKVLRTEK QYLGVYIWNMRGSDGTSTYATFLVTWKGDEKTRNPTPAVTPQPRGAEFHMWNYHSH VFSVGDTFSLAMHLQYKIHEAPFDLLLEWLYVPIDPTCQPMRLYSTCLYHPNAPQCLSH MNSGCTFTSPHLAQRVASTVYQNCEHADNYTAYCLGISHMEPSFGLILHDGGTTLKFV DTPESLSGLYVFVVYFNGHVEAVAYTVVSTVDHFVNAIEERGFPPTAGQPPATTKPKEI TPVNPGTSPLLRYAAWTGGLAAVVLLCLVIFLICTAKRMRVKAARVDKYPYDVPDYA GSSGVSGWRLFKKIS |
| 5 | VZVE-4 amino acid sequence | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQ PTQMSAQEDLGDDTGIHVIPTLNGDDRHKIVNVDQRQYGDVFKGDLNPKPQGQRLIEV SVEENHPFTLRAPIQRIYGVRYTETWSFLPSLTCTGDAAPAIQHICLKHTTCFQDVVVDV DCAENTKEDQLAEISYRFQGKKEADQPWIVVNTSTLFDELELDPPEIEPGVLKVLRTEK QYLGVYIWNMRGSDGTSTYATFLVTWKGDEKTRNPTPAVTPQPRGAEFHMWNYHSH VFSVGDTFSLAMHLQYKIHEAPFDLLLEWLYVPIDPTCQPMRLYSTCLYHPNAPQCLSH MNSGCTFTSPHLAQRVASTVYQNCEHADNYTAYCLGISHMEPSFGLILHDGGTTLKFV DTPESLSGLYVFVVYFNGHVEAVAYTVVSTVDHFVNAIEERGFPPTAGQPPATTKPKEI TPVNPGTSPLLRYAAWTGGLAAVVLLCLVIFLICTAKRMRVKAARVDKSPYNQSMYG AGLPVDDFEDAEAADAEEEFGNAIGGSHGGSSYTVYIDKTRYPYDVPDYAGSSGVSGW RLFKKIS |
| 6 | VZVE-5 amino acid sequence | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQ PTQMSAQEDLGDDTGIHVIPTLNGDDRHKIVNVDQRQYGDVFKGDLNPKPQGQRLIEV SVEENHPFTLRAPIQRIYGVRYTETWSFLPSLTCTGDAAPAIQHICLKHTTCFQDVVVDV DCAENTKEDQLAEISYRFQGKKEADQPWIVVNTSTLFDELELDPPEIEPGVLKVLRTEK QYLGVYIWNMRGSDGTSTYATFLVTWKGDEKTRNPTPAVTPQPRGAEFHMWNYHSH VFSVGDTFSLAMHLQYKIHEAPFDLLLEWLYVPIDPTCQPMRLYSTCLYHPNAPQCLSH MNSGCTFTSPHLAQRVASTVYQNCEHADNYTAYCLGISHMEPSFGLILHDGGTTLKFV DTPESLSGLYVFVVYFNGHVEAVAYTVVSTVDHFVNAIEERGFPPTAGQPPATTKPKEI TPVNPGTSPLLRYAAWTGGLAAVVLLCLVIFLICTAKRMRVKAYPYDVPDYAGSSGVS GWRLFKKIS |
| 7 | VZV gE-6 amino acid sequence | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQ PTQMSAQEDLGDDTGIHVIPTLNGDDRHKIVNVDQRQYGDVFKGDLNPKPQGQRLIEV SVEENHPFTLRAPIQRIYGVRYTETWSFLPSLTCTGDAAPAIQHICLKHTTCFQDVVVDV DCAENTKEDQLAEISYRFQGKKEADQPWIVVNTSTLFDELELDPPEIEPGVLKVLRTEK QYLGVYIWNMRGSDGTSTYATFLVTWKGDEKTRNPTPAVTPQPRGAEFHMWNYHSH VFSVGDTFSLAMHLQYKIHEAPFDLLLEWLYVPIDPTCQPMRLYSTCLYHPNAPQCLSH MNSGCTFTSPHLAQRVASTVYQNCEHADNYTAYCLGISHMEPSFGLILHDGGTTLKFV DTPESLSGLYVFVVYFNGHVEAVAYTVVSTVDHFVNAIEERGFPPTAGQPPATTKPKEI TPVNPGTSPLLRQILAIYSTVASSLVLVVSLGAISFWMCSNGSLQCRICI |
| 8 | VZV gE-7 amino acid sequence | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQ PTQMSAQEDLGDDTGIHVIPTLNGDDRHKIVNVDQRQYGDVFKGDLNPKPQGQRLIEV SVEENHPFTLRAPIQRIYGVRYTETWSFLPSLTCTGDAAPAIQHICLKHTTCFQDVVVDV DCAENTKEDQLAEISYRFQGKKEADQPWIVVNTSTLFDELELDPPEIEPGVLKVLRTEK QYLGVYIWNMRGSDGTSTYATFLVTWKGDEKTRNPTPAVTPQPRGAEFHMWNYHSH VFSVGDTFSLAMHLQYKIHEAPFDLLLEWLYVPIDPTCQPMRLYSTCLYHPNAPQCLSH MNSGCTFTSPHLAQRVASTVYQNCEHADNYTAYCLGISHMEPSFGLILHDGGTTLKFV DTPESLSGLYVFVVYFNGHVEAVAYTVVSTVDHFVNAIEERGFPPTAGQPPATTKPKEI TPVNPGTSPLLRWPWYIWLGFIAGLIAIVMVTIMLCCMTSCCSCLKGCCSCGSCCK |
| 9 | VZVE-4-deltag amino acid sequence | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQ PTQMSAQEDLGDDTGIHVIPTLNGDDRHKIVNVDQRQYGDVFKGDLNPKPQGQRLIEV SVEENHPFTLRAPIQRIYGVRYTETWSFLPSLTCTGDAAPAIQHICLKHTTCFQDVVVDV DCAENTKEDQLAEISYRFQGKKEADQPWIVVNTSTLFDELELDPPEIEPGVLKVLRTEK QYLGVYIWNMRGSDGTSTYATFLVTWKGDEKTRNPTPAVTPQPRGAEFHMWNYHSH VFSVGDTFSLAMHLQYKIHEAPFDLLLEWLYVPIDPTCQPMRLYSTCLYHPNAPQCLSH MNSGCTFTSPHLAQRVASTVYQNCEHADNYTAYCLGISHMEPSFGLILHDGGTTLKFV DTPESLSGLYVFVVYFNGHVEAVAYTVVSTVDHFVNAIEERGFPPTAGQPPATTKPKEI TPVNPGTSPLLRYAAWTGGLAAVVLLCLVIFLICTAKRMRVKAARVDKSPYNQSMYG AGLPVDDFEDAEAADAEEEFGNAIGGSHGGSSYTVYIDKTR |
| 10 | SARS-CoV-2 Spike protein transmem brane domain amino acid sequence | WPWYIWLGFIAGLIAIVMVTIMLCCMTSCCSCLKGCCSCGSCCK |
| 11 | Influenza H protein transmem brane domain amino acid sequence | QILAIYSTVASSLVLVVSLGAISFWMCSNGSLQCRICI |
| 12 | VZVE-1 mRNA sequence | AUGGGCACCGUGAAUAAGCCAGUGGUGGGCGUGCUGAUGGGCUUUGGCAUUAUC ACCGGAACACUGAGGAUCACCAACCCAGUGAGGGCCUCUGUGCUGAGAUACGAC GACUUCCACAUCGACGAGGACAAGCUGGACACCAACAGCGUGUACGAGCCAUAC UACCACAGCGAUCAUGCCGAGAGCUCUUGGGUGAACAGGGGAGAGAGCAGCAGG AAGGCCUACGACCACAAUUCUCCCUAUAUCUGGCCCAGGAACGACUACGAUGGCU UCCUGGAGAAUGCCCAUGAACACCACGGAGUGUACAACCAGGGCAGAGGCAUUG ACUCUGGAGAGAGGCUGAUGCAGCCAACCCAGAUGUCUGCCCAGGAGGAUCUGG GAGACGAUACCGGCAUCCAUGUGAUUCCCACACUGAACGGAGACGACAGGCACA AGAUCGUGAAUGUGGACCAGAGACAGUACGGCGAUGUGUUCAAGGGCGACCUGA AUCCUAAGCCUCAGGGCCAGCGACUGAUUGAGGUGUCUGUGGAGGAGAACCAUC CUUUCACACUGCGGGCUCCUAUCCAGCGGAUCUACGGCGUGAGAUACACCGAAAC CUGGAGCUUUCUGCCAAGCCUGACCUGUACAGGCGAUGCCGCUCCUGCUAUCCAG CACAUCUGCCUGAAGCAUACAACAUGCUUUCAGGACGUGGUGGUGGAUGUGGAU UGCGCCGAGAACACCAAAGAGGACCAGCUGGCCGAGAUUAGCUACAGAUUCCAG GGCAAGAAGGAGGCCGAUCAGCCCUGGAUCGUGGUGAAUACCUCUACCCUGUUU GACGAGCUGGAGCUGGACCCCCCUGAGAUUGAGCCUGGAGUGCUGAAGGUGCUG AGAACAGAGAAGCAGUACCUGGGCGUGUACAUUUGGAACAUGAGAGGCAGCGAC GGCACCUCUACCUAUGCCACAUUUCUGGUGACCUGGAAGGGCGACGAGAAAACC AGAAAUCCAACACCUGCCGUGACACCCCAGCCCAGGGGAGCCGAGUUUCACAUGU GGAAUUACCACAGCCACGUGUUCAGCGUGGGCGACACAUUUAGCCUGGCCAUGC ACCUGCAGUACAAAAUCCACGAGGCUCCUUUCGAUCUGCUGCUGGAGUGGCUGU AUGUGCCUAUUGACCCUACCUGCCAGCCUAUGAGGCUGUACAGCACCUGUCUGU ACCACCCAAACGCCCCUCAGUGCCUGAGCCAUAUGAACAGCGGCUGUACAUUCAC CUCUCCACACCUGGCCCAGAGAGUGGCCAGCACCGUGUAUCAGAAUUGCGAGCAU GCCGAUAACUACACAGCCUACUGUCUGGGCAUUAGCCACAUGGAGCCUAGCUUU GGCCUGAUCCUGCACGAUGGAGGCACCACCCUGAAAUUCGUGGAUACCCCCGAGA GCCUGAGCGGCCUGUACGUGUUUGUGGUGUACUUCAAUGGACACGUGGAGGCCG UGGCCUACACAGUGGUGAGCACAGUGGAUCACUUCGUGAACGCUAUUGAGGAGA GAGGAUUUCCUCCAACAGCCGGCCAGCCUCCAGCCACAACCAAACCCAAAGAAAU CACCCCCGUGAAUCCUGGAACAUCUCCCCUGCUGAGGUAUGCUGCUUGGACAGGA GGCCUGGCUGCCGUGGUGCUGCUGUGCCUGGUGAUCUUUCUGAUCUGUACCGCU AAGAGAAUGAGGGUGAAGGCCUAUAGAGUGGACAAGAGCCCCUACAACCAGAGC AUGUACUACGCCGGCCUGCCUGUGGACGAUUUUGAGGAUAGCGAGAGCACAGAU ACCGAGGAGGAGUUUGGCAAUGCCAUCGGCGGCAGCCAUGGCGGCAGCAGCUAC ACCGUGUACAUCGAUAAGACCAGGUAUCCAUACGAUGUGCCCGAUUAUGCCGGA UCUUCUGGAGUGUCUGGCUGGCGGCUGUUCAAGAAGAUCAGC |
| 13 | VZVE-2 mRNA sequence | AUGGGCACCGUGAACAAGCCUGUGGUGGGAGUGCUGAUGGGCUUCGGAAUCAUU ACAGGCACACUGAGAAUCACCAAUCCUGUGAGAGCCAGCGUGCUGCGGUACGAC GACUUCCACAUCGACGAGGACAAGCUGGACACCAACAGCGUGUACGAGCCCUACU ACCACAGCGACCACGCCGAGAGCAGCUGGGUGAAUAGGGGCGAAUCUAGCAGAA AAGCCUACGACCACAAUAGCCCUUACAUCUGGCCUAGGAACGAUUACGACGGAU UUCUGGAGAAUGCUCACGAGCACCACGGCGUGUACAAUCAGGGCAGGGGCAUUG ACUCUGGCGAGAGGCUGAUGCAGCCUACCCAGAUGAGCGCCCAGGAAGAUCUGG GCGACGACACAGGCAUCCAUGUGAUCCCUACACUGAAUGGAGACGACAGACACA AGAUCGUGAAUGUGGAUCAGAGACAGUACGGCGACGUGUUUAAAGGCGAUCUGA ACCCCAAGCCUCAGGGCCAGCGGCUGAUCGAGGUGUCUGUGGAGGAGAACCACCC UUUUACACUGCGGGCCCCUAUCCAGCGGAUCUACGGCGUGAGAUACACAGAGAC AUGGAGCUUCCUGCCUUCUCUGACCUGUACAGGAGACGCUGCUCCUGCCAUUCAG CACAUCUGCCUGAAACACACCACAUGCUUUCAGGACGUGGUGGUGGACGUGGAU UGUGCCGAGAAUACCAAGGAGGACCAGCUGGCCGAGAUCAGCUACAGGUUCCAG GGAAAGAAGGAGGCCGACCAGCCUUGGAUUGUGGUGAACACCUCUACCCUGUUU GAUGAGCUGGAGCUGGACCCUCCAGAGAUUGAGCCCGGCGUGCUGAAAGUGCUG AGAACCGAGAAGCAGUAUCUGGGCGUGUAUAUCUGGAACAUGAGGGGCAGCGAU GGAACCAGCACCUACGCCACCUUCCUGGUGACAUGGAAGGGCGACGAGAAGACA AGGAAUCCUACACCAGCCGUGACACCUCAGCCAAGAGGCGCCGAGUUUCACAUGU GGAAUUAUCACUCUCACGUGUUCAGCGUGGGCGACACCUUCAGCCUGGCCAUGC AUCUGCAGUAUAAGAUCCACGAGGCCCCCUUUGAUCUGCUGCUGGAGUGGCUGU AUGUGCCUAUCGACCCAACCUGCCAGCCAAUGAGGCUGUACUCUACCUGCCUGUA CCACCCAAACGCCCCUCAGUGCCUGAGCCACAUGAACAGCGGCUGUACAUUUACA AGCCCCCAUCUUGCUCAGAGGGUGGCCUCUACAGUGUACCAGAAUUGCGAGCAC GCCGAUAAUUACACAGCCUACUGCCUGGGCAUCAGCCACAUGGAGCCUAGCUUU GGCCUGAUCCUGCACGAUGGCGGCACCACCCUGAAGUUUGUGGAUACACCUGAG AGCCUGAGCGGCCUGUACGUGUUCGUGGUGUACUUUAACGGACAUGUGGAGGCC GUGGCCUAUACAGUGGUGAGCACCGUGGACCAUUUUGUGAAUGCCAUUGAGGAG AGAGGAUUCCCUCCUACAGCCGGACAGCCUCCAGCCACCACCAAGCCAAAGGAAA UCACACCCGUGAAUCCAGGAACCUCUCCUCUGCUGAGAUAUGCUGCCUGGACAGG CGGCCUGGCUGCUGUGGUGCUGCUGUGUCUGGUUAUCUUCCUGAUCUGUACAGC UAAGAGGAUGCGGGUGAAGGCCUACAGGGUGGAUAAGAGCCCAUACAACCAGAG CAUGUACUACGCCGGCCUGCCUGUGGAUGAUUUCGAGGAUAGCGAAAGCACCGA UACCGAGGAGGAGUUUGGCAACGCCAUUGGCGGCUCUCACGGCGGCAGCAGCUA CACCGUGUAUAUUGACAAGACCAGGUACCCUUACGAUGUGCCAGAUUACGCCGG AAGCUCUGGCGUGUCUGGCUGGCGGCUGUUCAAGAAGAUCAGC |
| 14 | VZVE-3 mRNA sequence | AUGGGCACCGUGAAUAAGCCCGUGGUGGGCGUGCUGAUGGGCUUUGGCAUCAUC ACAGGAACACUGAGAAUCACCAACCCUGUGAGAGCCAGCGUGCUGAGAUACGAC GACUUUCACAUCGACGAGGACAAGCUGGACACCAACAGCGUGUACGAGCCUUAC UACCACAGCGACCACGCCGAGAGCAGCUGGGUGAAUAGGGGAGAGAGCAGCAGG AAGGCCUACGACCACAAUAGCCCAUACAUCUGGCCCAGAAACGAUUACGAUGGC UUUCUGGAAAACGCCCACGAGCAUCACGGAGUGUACAAUCAGGGAAGGGGCAUU GAUAGCGGCGAAAGGCUGAUGCAGCCCACCCAGAUGUCUGCCCAGGAGGAUCUG GGAGAUGAUACCGGCAUUCACGUGAUCCCCACCCUGAAUGGCGACGACAGGCAC AAGAUCGUGAACGUGGACCAGAGACAGUAUGGCGACGUGUUCAAAGGAGAUCUG AACCCUAAGCCUCAGGGACAGAGGCUGAUUGAGGUGAGCGUGGAGGAGAACCAC CCUUUUACCCUGCGGGCCCCUAUCCAGAGAAUCUACGGCGUGAGAUACACCGAAA CAUGGAGCUUCCUGCCUUCUCUGACAUGCACAGGCGAUGCCGCCCCUGCCAUCCA GCACAUCUGCCUGAAGCACACAACAUGCUUUCAGGAUGUGGUGGUGGAUGUGGA UUGUGCCGAGAACACAAAGGAGGACCAGCUGGCCGAGAUCUCUUACAGAUUCCA GGGCAAGAAGGAGGCCGACCAGCCUUGGAUCGUGGUGAAUACCUCUACCCUGUU CGAUGAGCUGGAGCUGGACCCACCAGAGAUCGAGCCUGGCGUGCUCAAGGUGCU GAGAACCGAGAAACAGUACCUGGGCGUGUACAUUUGGAACAUGAGAGGCUCUGA CGGCACAUCUACCUACGCCACCUUUCUGGUGACCUGGAAGGGAGACGAGAAGAC CAGAAACCCAACACCUGCCGUGACACCCCAGCCAAGAGGCGCCGAGUUUCACAUG UGGAAUUAUCACAGCCACGUGUUCAGCGUGGGCGACACCUUCAGCCUGGCUAUG CAUCUGCAGUAUAAGAUCCACGAAGCCCCAUUCGACCUGCUGCUGGAGUGGCUG UAUGUGCCAAUCGACCCAACAUGCCAGCCCAUGCGCCUGUACAGCACCUGUCUGU AUCAUCCUAACGCCCCACAGUGCUUAAGCCACAUGAACAGCGGCUGUACAUUCAC AUCUCCACACCUCGCCCAGAGGGUGGCCAGCACCGUGUACCAGAAUUGUGAACAU GCCGACAAUUACACCGCCUACUGCCUGGGAAUCAGCCACAUGGAGCCUAGCUUCG GCCUGAUCCUGCACGAUGGAGGCACAACCCUGAAGUUUGUGGACACACCCGAGU CACUGAGCGGCCUGUACGUGUUUGUGGUGUACUUCAAUGGACACGUGGAGGCCG UGGCCUACACAGUGGUGUCUACCGUGGAUCAUUUCGUGAACGCUAUCGAGGAGA GAGGAUUUCCUCCUACAGCUGGACAGCCUCCCGCCACCACCAAACCCAAGGAGAU UACACCUGUGAAUCCAGGCACAUCUCCCCUGCUGCGGUAUGCUGCUUGGACAGG AGGCCUGGCUGCUGUGGUGCUGCUGUGUCUGGUGAUCUUCCUGAUCUGCACCGC UAAAAGAAUGAGGGUGAAAGCCUACCGGGUGGAUAAAAGCCCUUACAACCAGAG CAUGUACUACGCCGGCCUGCCCGUGGAUGACUUUGAAGACAGCGAGAGCACCGA UACCGAAGAGGAGUUUGGCAAUGCCAUCGGCGGCUCCCACGGCGGCAGCAGCUA UACCGUGUACAUCGACAAGACCAGAUACCCUUACGAUGUCCCUGACUAUGCCGGC UCUAGCGGAGUGUCUGGCUGGAGAUUGUUCAAGAAGAUCAGC |
| 15 | VZVE-1-1 mRNA sequence | AUGGGCACCGUGAACAAGCCUGUGGUGGGCGUGCUGAUGGGAUUUGGAAUCAUC ACCGGCACCCUGAGAAUCACCAACCCUGUGAGAGCCAGCGUGCUGAGGUAUGAC GACUUCCAUAUCGACGAGGACAAGCUGGACACCAAUAGCGUGUACGAGCCCUAC UACCACAGCGACCACGCCGAGAGCUCUUGGGUGAAUAGAGGAGAGAGCAGCAGG AAGGCCUACGAUCACAACAGCCCUUACAUCUGGCCCAGAAACGACUAUGACGGCU UCCUGGAAAACGCCCACGAGCACCAUGGCGUGUAUAAUCAGGGCAGAGGAAUCG AUAGCGGAGAGAGGCUGAUGCAACCUACCCAGAUGUCUGCCCAGGAGGAUCUGG GCGAUGAUACCGGAAUCCACGUGAUCCCUACCCUGAACGGCGACGACAGACACAA GAUCGUGAAUGUGGACCAGAGACAGUACGGCGAUGUGUUCAAGGGCGAUCUGAA CCCCAAACCUCAGGGACAGAGGCUCAUCGAGGUGAGCGUGGAGGAGAACCAUCC UUUUACCCUGAGGGCCCCUAUCCAGAGAAUCUACGGCGUGAGGUAUACCGAGAC AUGGUCCUUCCUGCCCAGCCUGACCUGUACAGGCGAUGCCGCUCCUGCCAUCCAG CAUAUUUGCCUGAAGCAUACCACCUGUUUUCAGGAUGUGGUGGUGGAUGUGGAU UGCGCCGAGAACACCAAGGAGGACCAGCUGGCCGAGAUCUCUUAUAGAUUCCAG GGCAAGAAGGAGGCCGACCAGCCUUGGAUUGUGGUGAACACCUCUACCCUGUUC GACGAGCUGGAGCUGGACCCCCCUGAGAUUGAACCAGGCGUUCUGAAGGUGCUG AGAACCGAGAAGCAGUACCUGGGAGUGUACAUCUGGAAUAUGAGAGGCAGCGAU GGCACCAGCACCUACGCUACCUUUCUGGUGACCUGGAAGGGAGACGAGAAAACC AGGAAUCCAACCCCUGCCGUGACACCACAGCCCAGAGGCGCCGAGUUUCACAUGU GGAAUUACCACAGCCACGUGUUCAGCGUGGGCGACACCUUCUCUCUGGCCAUGCA CCUGCAGUAUAAGAUUCACGAAGCUCCAUUUGACCUGCUGCUGGAGUGGCUGUA CGUGCCAAUUGACCCCACAUGCCAGCCAAUGAGGCUGUACAGCACCUGCCUGUAC CAUCCAAACGCCCCCCAGUGCCUGUCUCACAUGAACAGCGGCUGUACAUUUACAA GCCCUCACCUGGCCCAGCGAGUGGCCAGCACAGUGUACCAGAAUUGUGAGCAUGC UGACAAUUACACAGCCUAUUGCCUGGGCAUCAGCCACAUGGAGCCAUCUUUCGG CCUGAUCCUGCACGAUGGCGGCACCACCCUGAAAUUUGUGGAUACCCCUGAGAGC CUGAGCGGCCUGUACGUGUUUGUGGUGUACUUUAACGGCCACGUGGAGGCCGUG GCCUACACCGUGGUGAGCACAGUGGAUCAUUUUGUGAAUGCCAUCGAAGAAAGA GGCUUCCCUCCUACAGCCGGACAGCCUCCUGCCACAACAAAGCCCAAGGAGAUUA CCCCAGUGAAUCCAGGAACCUCUCCACUGCUGAGAUACGCCGCUUGGACAGGAGG CCUGGCUGCUGUGGUGCUGCUGUGCCUGGUGAUCUUCCUGAUUUGCACCGCUAA GAGAAUGAGAGUGAAAGCCGCCAGAGUGGAUAAGAGCCCCUACAACCAGAGCAU GUACUAUGCCGGCCUGCCUGUGGAUGAUUUCGAAGAUGCCGAAGCUGCCGAUGC UGAGGAGGAGUUUGGCAAUGCCAUUGGCGGCAGCCACGGCGGCAGCAGCUAUAC CGUGUACAUCGACAAGACAAGGUACCCUUACGACGUGCCCGAUUAUGCUGGAAG CUCUGGAGUGUCUGGCUGGAGGCUGUUCAAGAAGAUCAGC |
| 16 | VZVE-1-2 mRNA sequence | AUGGGCACCGUGAACAAGCCUGUGGUGGGCGUGCUCAUGGGCUUUGGCAUCAUC ACAGGCACCCUGAGAAUCACCAAUCCCGUGAGAGCCAGCGUGCUGCGGUACGACG ACUUCCACAUCGACGAGGACAAGCUGGACACCAAUAGCGUGUACGAGCCCUACU ACCACAGCGACCACGCCGAGAGCAGCUGGGUGAAUAGAGGAGAAUCUAGCAGGA AGGCCUAUGACCACAAUAGCCCAUACAUUUGGCCCAGGAAUGAUUACGACGGCU UCCUGGAGAACGCCCACGAGCACCACGGCGUGUACAACCAGGGAAGAGGCAUUG AUUCUGGCGAACGGCUGAUGCAGCCAACACAGAUGAGCGCCCAGGAGGACCUGG GAGAUGAUACCGGAAUCCACGUGAUUCCUACCCUGAACGGCGAUGAUAGACACA AGAUCGUGAACGUGGACCAGAGACAGUACGGCGACGUGUUUAAGGGCGACCUGA ACCCCAAGCCUCAGGGACAGAGGCUGAUUGAGGUGAGCGUGGAAGAGAACCACC CUUUCACACUGAGAGCCCCCAUCCAGAGGAUUUACGGCGUGAGGUACACAGAGA CAUGGAGCUUUCUGCCUAGCCUGACAUGUACAGGCGAUGCCGCCCCUGCCAUUCA GCACAUCUGCCUGAAGCACACCACCUGUUUUCAGGAUGUGGUGGUGGAUGUGGA UUGCGCCGAGAACACCAAGGAGGAUCAGCUGGCCGAGAUCAGCUACAGAUUCCA GGGAAAGAAGGAGGCCGACCAGCCUUGGAUCGUGGUGAAUACAAGCACCCUGUU CGAUGAGCUGGAGCUGGACCCUCCCGAGAUUGAACCUGGCGUGCUGAAGGUGCU GAGGACCGAGAAGCAGUAUCUGGGAGUGUACAUUUGGAAUAUGAGGGGCAGCGA CGGCACAUCUACCUACGCCACCUUCCUGGUGACAUGGAAGGGCGACGAAAAAACC CGGAAUCCUACCCCUGCCGUGACCCCACAGCCUAGAGGAGCCGAGUUUCACAUGU GGAAUUACCACAGCCACGUGUUCAGCGUGGGAGACACAUUCUCUCUGGCCAUGC ACCUGCAGUACAAGAUUCACGAAGCCCCAUUCGACCUGCUGCUGGAGUGGCUGU AUGUGCCCAUUGAUCCCACCUGUCAGCCCAUGCGACUGUACAGCACCUGUCUGUA UCACCCAAACGCCCCCCAGUGUCUGUCUCACAUGAAUAGCGGAUGCACCUUUACA AGCCCACAUCUGGCCCAGAGGGUGGCUAGCACAGUGUACCAGAAUUGCGAGCAC GCCGACAACUACACAGCUUACUGCCUGGGCAUCAGCCACAUGGAGCCUUCUUUCG GCCUGAUCCUGCACGACGGCGGCACAACACUGAAAUUUGUGGAUACCCCAGAGA GCCUGUCUGGCCUGUACGUGUUUGUGGUGUAUUUUAACGGCCACGUGGAGGCCG UGGCCUACACAGUGGUGUCUACCGUGGACCACUUUGUGAACGCUAUCGAGGAGA GAGGCUUUCCUCCAACAGCUGGACAGCCUCCAGCUACCACAAAGCCUAAGGAGAU CACCCCUGUGAACCCCGGAACAUCUCCACUGCUGAGAUACGCCGCUUGGACCGGC GGAUUGGCUGCCGUUGUGCUGCUGUGCCUGGUGAUCUUUCUGAUUUGUACAGCC AAGAGAAUGAGAGUGAAGGCUGCUCGGGUGGACAAGAGCCCUUACAAUCAGAGC AUGUACUACGCCGGCCUGCCUGUGGAUGACUUCGAAGAUGCCGAAGCUGCCGAU GCCGAGGAGGAAUUUGGCAAUGCUAUCGGCGGCAGCCAUGGCGGCAGCAGCUAC ACCGUGUACAUCGACAAAACCAGGUAUCCAUACGAUGUGCCCGAUUAUGCCGGA UCUUCUGGCGUGAGCGGCUGGAGAUUGUUCAAGAAGAUCUCC |
| 17 | VZVE-1-3 mRNA sequence | AUGGGCACCGUGAACAAGCCUGUGGUGGGAGUGCUCAUGGGAUUUGGAAUCAUU ACAGGCACACUGAGGAUCACCAACCCCGUGAGAGCCAGCGUGCUGAGGUACGAU GACUUCCACAUCGACGAGGACAAGCUGGACACCAACAGCGUGUACGAGCCCUACU ACCACAGCGACCACGCCGAGAGCUCUUGGGUGAAUAGGGGCGAAUCUAGCAGAA AGGCCUACGACCACAAUAGCCCCUAUAUUUGGCCUAGAAACGACUACGAUGGCU UCCUGGAGAACGCCCACGAGCACCACGGAGUGUACAAUCAGGGCAGAGGCAUCG AUAGCGGCGAAAGGCUGAUGCAGCCUACCCAGAUGAGCGCCCAGGAAGAUCUGG GCGAUGACACAGGCAUCCAUGUGAUUCCUACACUGAACGGCGACGACAGGCACA AAAUCGUGAAUGUGGAUCAGAGGCAGUACGGCGACGUGUUUAAGGGCGAUCUGA AUCCCAAGCCACAGGGCCAGAGGCUGAUCGAAGUGAGCGUGGAGGAGAAUCACC CUUUUACCCUGCGGGCCCCAAUCCAGCGGAUCUACGGAGUGAGGUAUACCGAAA CAUGGAGCUUUCUGCCAAGCCUGACCUGUACCGGAGACGCUGCCCCUGCUAUCCA GCACAUUUGUCUGAAGCACACCACCUGUUUUCAGGACGUGGUGGUGGAUGUGGA UUGUGCCGAGAACACCAAGGAGGACCAGCUGGCCGAGAUCAGCUACAGAUUCCA GGGCAAGAAGGAGGCCGAUCAGCCAUGGAUUGUUGUGAAUACCAGCACCCUGUU CGACGAGCUGGAGCUGGACCCUCCAGAAAUUGAGCCUGGCGUGCUGAAGGUGCU GAGAACCGAGAAACAGUACCUGGGAGUUUACAUCUGGAACAUGAGGGGCUCCGA CGGCACAAGCACCUACGCUACCUUCCUGGUGACAUGGAAGGGCGACGAGAAGAC AAGAAAUCCUACCCCAGCCGUGACACCACAGCCCAGGGGCGCCGAGUUUCAUAUG UGGAAUUACCACAGCCACGUGUUCAGCGUGGGCGAUACAUUUAGCCUGGCCAUG CAUCUGCAGUACAAGAUCCACGAGGCUCCUUUCGACCUGCUGCUGGAAUGGCUG UACGUGCCAAUCGACCCCACAUGCCAGCCUAUGAGGCUGUAUAGCACAUGCCUGU AUCACCCUAACGCCCCCCAGUGCCUGAGCCACAUGAACAGCGGAUGCACAUUUAC CUCUCCUCACCUGGCCCAGAGAGUGGCCAGCACAGUGUAUCAGAAUUGCGAGCAC GCCGACAAUUAUACUGCCUACUGCCUGGGCAUUAGCCACAUGGAGCCCAGCUUU GGCCUGAUCCUGCAUGAUGGCGGCACAACACUGAAAUUCGUGGAUACCCCCGAG AGCCUGUCUGGCCUGUACGUGUUCGUGGUGUACUUUAAUGGCCACGUGGAGGCC GUGGCCUACACAGUGGUUAGCACAGUGGAUCACUUCGUGAACGCCAUCGAAGAG AGAGGCUUUCCACCUACAGCCGGACAGCCUCCUGCUACCACAAAACCCAAGGAGA UCACCCCUGUGAAUCCUGGAACCAGCCCACUGCUGAGAUAUGCUGCUUGGACAG GCGGCCUGGCUGCUGUGGUGCUGCUGUGUCUGGUGAUCUUUCUGAUCUGUACCG CUAAGAGGAUGAGGGUGAAGGCCGCCAGAGUGGAUAAAAGCCCAUACAACCAGA GCAUGUACUACGCCGGCCUGCCUGUGGAUGAUUUUGAGGAUGCUGAAGCCGCCG AUGCCGAAGAGGAGUUUGGCAAUGCCAUUGGCGGAAGCCACGGCGGCAGCAGCU ACACCGUGUACAUCGACAAGACAAGGUAUCCCUACGAUGUGCCUGAUUAUGCCG GAAGCUCUGGCGUGUCUGGAUGGAGGCUGUUCAAGAAGAUCAGC |
| 18 | VZVE-2-1 mRNA sequence | AUGGGCACCGUGAAUAAGCCAGUGGUGGGCGUGCUGAUGGGCUUUGGCAUUAUC ACCGGAACACUGAGGAUCACCAACCCAGUGAGGGCCUCUGUGCUGAGAUACGAC GACUUCCACAUCGACGAGGACAAGCUGGACACCAACAGCGUGUACGAGCCAUAC UACCACAGCGAUCAUGCCGAGAGCUCUUGGGUGAACAGGGGAGAGAGCAGCAGG AAGGCCUACGACCACAAUUCUCCCUAUAUCUGGCCCAGGAACGACUACGAUGGCU UCCUGGAGAAUGCCCAUGAACACCACGGAGUGUACAACCAGGGCAGAGGCAUUG ACUCUGGAGAGAGGCUGAUGCAGCCAACCCAGAUGUCUGCCCAGGAGGAUCUGG GAGACGAUACCGGCAUCCAUGUGAUUCCCACACUGAACGGAGACGACAGGCACA AGAUCGUGAAUGUGGACCAGAGACAGUACGGCGAUGUGUUCAAGGGCGACCUGA AUCCUAAGCCUCAGGGCCAGCGACUGAUUGAGGUGUCUGUGGAGGAGAACCAUC CUUUCACACUGCGGGCUCCUAUCCAGCGGAUCUACGGCGUGAGAUACACCGAAAC CUGGAGCUUUCUGCCAAGCCUGACCUGUACAGGCGAUGCCGCUCCUGCUAUCCAG CACAUCUGCCUGAAGCAUACAACAUGCUUUCAGGACGUGGUGGUGGAUGUGGAU UGCGCCGAGAACACCAAAGAGGACCAGCUGGCCGAGAUUAGCUACAGAUUCCAG GGCAAGAAGGAGGCCGAUCAGCCCUGGAUCGUGGUGAAUACCUCUACCCUGUUU GACGAGCUGGAGCUGGACCCCCCUGAGAUUGAGCCUGGAGUGCUGAAGGUGCUG AGAACAGAGAAGCAGUACCUGGGCGUGUACAUUUGGAACAUGAGAGGCAGCGAC GGCACCUCUACCUAUGCCACAUUUCUGGUGACCUGGAAGGGCGACGAGAAAACC AGAAAUCCAACACCUGCCGUGACACCCCAGCCCAGGGGAGCCGAGUUUCACAUGU GGAAUUACCACAGCCACGUGUUCAGCGUGGGCGACACAUUUAGCCUGGCCAUGC ACCUGCAGUACAAAAUCCACGAGGCUCCUUUCGAUCUGCUGCUGGAGUGGCUGU AUGUGCCUAUUGACCCUACCUGCCAGCCUAUGAGGCUGUACAGCACCUGUCUGU ACCACCCAAACGCCCCUCAGUGCCUGAGCCAUAUGAACAGCGGCUGUACAUUCAC CUCUCCACACCUGGCCCAGAGAGUGGCCAGCACCGUGUAUCAGAAUUGCGAGCAU GCCGAUAACUACACAGCCUACUGUCUGGGCAUUAGCCACAUGGAGCCUAGCUUU GGCCUGAUCCUGCACGAUGGAGGCACCACCCUGAAAUUCGUGGAUACCCCCGAGA GCCUGAGCGGCCUGUACGUGUUUGUGGUGUACUUCAAUGGACACGUGGAGGCCG UGGCCUACACAGUGGUGAGCACAGUGGAUCACUUCGUGAACGCUAUUGAGGAGA GAGGAUUUCCUCCAACAGCCGGCCAGCCUCCAGCCACAACCAAACCCAAAGAAAU CACCCCCGUGAAUCCUGGAACAUCUCCCCUGCUGAGGUAUGCUGCUUGGACAGGA GGCCUGGCUGCCGUGGUGCUGCUGUGCCUGGUGAUCUUUCUGAUCUGUACCUAU CCAUACGAUGUGCCCGAUUAUGCCGGAUCUUCUGGAGUGUCUGGCUGGCGGCUG UUCAAGAAGAUCAGC |
| 19 | VZVE-2-2 mRNA sequence | AUGGGCACCGUGAACAAGCCUGUGGUGGGAGUGCUGAUGGGCUUCGGAAUCAUU ACAGGCACACUGAGAAUCACCAAUCCUGUGAGAGCCAGCGUGCUGCGGUACGAC GACUUCCACAUCGACGAGGACAAGCUGGACACCAACAGCGUGUACGAGCCCUACU ACCACAGCGACCACGCCGAGAGCAGCUGGGUGAAUAGGGGCGAAUCUAGCAGAA AAGCCUACGACCACAAUAGCCCUUACAUCUGGCCUAGGAACGAUUACGACGGAU UUCUGGAGAAUGCUCACGAGCACCACGGCGUGUACAAUCAGGGCAGGGGCAUUG ACUCUGGCGAGAGGCUGAUGCAGCCUACCCAGAUGAGCGCCCAGGAAGAUCUGG GCGACGACACAGGCAUCCAUGUGAUCCCUACACUGAAUGGAGACGACAGACACA AGAUCGUGAAUGUGGAUCAGAGACAGUACGGCGACGUGUUUAAAGGCGAUCUGA ACCCCAAGCCUCAGGGCCAGCGGCUGAUCGAGGUGUCUGUGGAGGAGAACCACCC UUUUACACUGCGGGCCCCUAUCCAGCGGAUCUACGGCGUGAGAUACACAGAGAC AUGGAGCUUCCUGCCUUCUCUGACCUGUACAGGAGACGCUGCUCCUGCCAUUCAG CACAUCUGCCUGAAACACACCACAUGCUUUCAGGACGUGGUGGUGGACGUGGAU UGUGCCGAGAAUACCAAGGAGGACCAGCUGGCCGAGAUCAGCUACAGGUUCCAG GGAAAGAAGGAGGCCGACCAGCCUUGGAUUGUGGUGAACACCUCUACCCUGUUU GAUGAGCUGGAGCUGGACCCUCCAGAGAUUGAGCCCGGCGUGCUGAAAGUGCUG AGAACCGAGAAGCAGUAUCUGGGCGUGUAUAUCUGGAACAUGAGGGGCAGCGAU GGAACCAGCACCUACGCCACCUUCCUGGUGACAUGGAAGGGCGACGAGAAGACA AGGAAUCCUACACCAGCCGUGACACCUCAGCCAAGAGGCGCCGAGUUUCACAUGU GGAAUUAUCACUCUCACGUGUUCAGCGUGGGCGACACCUUCAGCCUGGCCAUGC AUCUGCAGUAUAAGAUCCACGAGGCCCCCUUUGAUCUGCUGCUGGAGUGGCUGU AUGUGCCUAUCGACCCAACCUGCCAGCCAAUGAGGCUGUACUCUACCUGCCUGUA CCACCCAAACGCCCCUCAGUGCCUGAGCCACAUGAACAGCGGCUGUACAUUUACA AGCCCCCAUCUUGCUCAGAGGGUGGCCUCUACAGUGUACCAGAAUUGCGAGCAC GCCGAUAAUUACACAGCCUACUGCCUGGGCAUCAGCCACAUGGAGCCUAGCUUU GGCCUGAUCCUGCACGAUGGCGGCACCACCCUGAAGUUUGUGGAUACACCUGAG AGCCUGAGCGGCCUGUACGUGUUCGUGGUGUACUUUAACGGACAUGUGGAGGCC GUGGCCUAUACAGUGGUGAGCACCGUGGACCAUUUUGUGAAUGCCAUUGAGGAG AGAGGAUUCCCUCCUACAGCCGGACAGCCUCCAGCCACCACCAAGCCAAAGGAAA UCACACCCGUGAAUCCAGGAACCUCUCCUCUGCUGAGAUAUGCUGCCUGGACAGG CGGCCUGGCUGCUGUGGUGCUGCUGUGUCUGGUUAUCUUCCUGAUCUGUACAUA CCCUUACGAUGUGCCAGAUUACGCCGGAAGCUCUGGCGUGUCUGGCUGGCGGCU GUUCAAGAAGAUCAGC |
| 20 | VZVE-2-3 mRNA sequence | AUGGGCACCGUGAAUAAGCCCGUGGUGGGCGUGCUGAUGGGCUUUGGCAUCAUC ACAGGAACACUGAGAAUCACCAACCCUGUGAGAGCCAGCGUGCUGAGAUACGAC GACUUUCACAUCGACGAGGACAAGCUGGACACCAACAGCGUGUACGAGCCUUAC UACCACAGCGACCACGCCGAGAGCAGCUGGGUGAAUAGGGGAGAGAGCAGCAGG AAGGCCUACGACCACAAUAGCCCAUACAUCUGGCCCAGAAACGAUUACGAUGGC UUUCUGGAAAACGCCCACGAGCAUCACGGAGUGUACAAUCAGGGAAGGGGCAUU GAUAGCGGCGAAAGGCUGAUGCAGCCCACCCAGAUGUCUGCCCAGGAGGAUCUG GGAGAUGAUACCGGCAUUCACGUGAUCCCCACCCUGAAUGGCGACGACAGGCAC AAGAUCGUGAACGUGGACCAGAGACAGUAUGGCGACGUGUUCAAAGGAGAUCUG AACCCUAAGCCUCAGGGACAGAGGCUGAUUGAGGUGAGCGUGGAGGAGAACCAC CCUUUUACCCUGCGGGCCCCUAUCCAGAGAAUCUACGGCGUGAGAUACACCGAAA CAUGGAGCUUCCUGCCUUCUCUGACAUGCACAGGCGAUGCCGCCCCUGCCAUCCA GCACAUCUGCCUGAAGCACACAACAUGCUUUCAGGAUGUGGUGGUGGAUGUGGA UUGUGCCGAGAACACAAAGGAGGACCAGCUGGCCGAGAUCUCUUACAGAUUCCA GGGCAAGAAGGAGGCCGACCAGCCUUGGAUCGUGGUGAAUACCUCUACCCUGUU CGAUGAGCUGGAGCUGGACCCACCAGAGAUCGAGCCUGGCGUGCUCAAGGUGCU GAGAACCGAGAAACAGUACCUGGGCGUGUACAUUUGGAACAUGAGAGGCUCUGA CGGCACAUCUACCUACGCCACCUUUCUGGUGACCUGGAAGGGAGACGAGAAGAC CAGAAACCCAACACCUGCCGUGACACCCCAGCCAAGAGGCGCCGAGUUUCACAUG UGGAAUUAUCACAGCCACGUGUUCAGCGUGGGCGACACCUUCAGCCUGGCUAUG CAUCUGCAGUAUAAGAUCCACGAAGCCCCAUUCGACCUGCUGCUGGAGUGGCUG UAUGUGCCAAUCGACCCAACAUGCCAGCCCAUGCGCCUGUACAGCACCUGUCUGU AUCAUCCUAACGCCCCACAGUGCUUAAGCCACAUGAACAGCGGCUGUACAUUCAC AUCUCCACACCUCGCCCAGAGGGUGGCCAGCACCGUGUACCAGAAUUGUGAACAU GCCGACAAUUACACCGCCUACUGCCUGGGAAUCAGCCACAUGGAGCCUAGCUUCG GCCUGAUCCUGCACGAUGGAGGCACAACCCUGAAGUUUGUGGACACACCCGAGU CACUGAGCGGCCUGUACGUGUUUGUGGUGUACUUCAAUGGACACGUGGAGGCCG UGGCCUACACAGUGGUGUCUACCGUGGAUCAUUUCGUGAACGCUAUCGAGGAGA GAGGAUUUCCUCCUACAGCUGGACAGCCUCCCGCCACCACCAAACCCAAGGAGAU UACACCUGUGAAUCCAGGCACAUCUCCCCUGCUGCGGUAUGCUGCUUGGACAGG AGGCCUGGCUGCUGUGGUGCUGCUGUGUCUGGUGAUCUUCCUGAUCUGCACCUA CCCUUACGAUGUCCCUGACUAUGCCGGCUCUAGCGGAGUGUCUGGCUGGAGAUU GUUCAAGAAGAUCAGC |
| 21 | VZVE-3-1 mRNA sequence | AUGGGCACCGUGAACAAGCCUGUGGUGGGCGUGCUGAUGGGAUUUGGAAUCAUC ACCGGCACCCUGAGAAUCACCAACCCUGUGAGAGCCAGCGUGCUGAGGUAUGAC GACUUCCAUAUCGACGAGGACAAGCUGGACACCAAUAGCGUGUACGAGCCCUAC UACCACAGCGACCACGCCGAGAGCUCUUGGGUGAAUAGAGGAGAGAGCAGCAGG AAGGCCUACGAUCACAACAGCCCUUACAUCUGGCCCAGAAACGACUAUGACGGCU UCCUGGAAAACGCCCACGAGCACCAUGGCGUGUAUAAUCAGGGCAGAGGAAUCG AUAGCGGAGAGAGGCUGAUGCAACCUACCCAGAUGUCUGCCCAGGAGGAUCUGG GCGAUGAUACCGGAAUCCACGUGAUCCCUACCCUGAACGGCGACGACAGACACAA GAUCGUGAAUGUGGACCAGAGACAGUACGGCGAUGUGUUCAAGGGCGAUCUGAA CCCCAAACCUCAGGGACAGAGGCUCAUCGAGGUGAGCGUGGAGGAGAACCAUCC UUUUACCCUGAGGGCCCCUAUCCAGAGAAUCUACGGCGUGAGGUAUACCGAGAC AUGGUCCUUCCUGCCCAGCCUGACCUGUACAGGCGAUGCCGCUCCUGCCAUCCAG CAUAUUUGCCUGAAGCAUACCACCUGUUUUCAGGAUGUGGUGGUGGAUGUGGAU UGCGCCGAGAACACCAAGGAGGACCAGCUGGCCGAGAUCUCUUAUAGAUUCCAG GGCAAGAAGGAGGCCGACCAGCCUUGGAUUGUGGUGAACACCUCUACCCUGUUC GACGAGCUGGAGCUGGACCCCCCUGAGAUUGAACCAGGCGUUCUGAAGGUGCUG AGAACCGAGAAGCAGUACCUGGGAGUGUACAUCUGGAAUAUGAGAGGCAGCGAU GGCACCAGCACCUACGCUACCUUUCUGGUGACCUGGAAGGGAGACGAGAAAACC AGGAAUCCAACCCCUGCCGUGACACCACAGCCCAGAGGCGCCGAGUUUCACAUGU GGAAUUACCACAGCCACGUGUUCAGCGUGGGCGACACCUUCUCUCUGGCCAUGCA CCUGCAGUAUAAGAUUCACGAAGCUCCAUUUGACCUGCUGCUGGAGUGGCUGUA CGUGCCAAUUGACCCCACAUGCCAGCCAAUGAGGCUGUACAGCACCUGCCUGUAC CAUCCAAACGCCCCCCAGUGCCUGUCUCACAUGAACAGCGGCUGUACAUUUACAA GCCCUCACCUGGCCCAGCGAGUGGCCAGCACAGUGUACCAGAAUUGUGAGCAUGC UGACAAUUACACAGCCUAUUGCCUGGGCAUCAGCCACAUGGAGCCAUCUUUCGG CCUGAUCCUGCACGAUGGCGGCACCACCCUGAAAUUUGUGGAUACCCCUGAGAGC CUGAGCGGCCUGUACGUGUUUGUGGUGUACUUUAACGGCCACGUGGAGGCCGUG GCCUACACCGUGGUGAGCACAGUGGAUCAUUUUGUGAAUGCCAUCGAAGAAAGA GGCUUCCCUCCUACAGCCGGACAGCCUCCUGCCACAACAAAGCCCAAGGAGAUUA CCCCAGUGAAUCCAGGAACCUCUCCACUGCUGAGAUACGCCGCUUGGACAGGAGG CCUGGCUGCUGUGGUGCUGCUGUGCCUGGUGAUCUUCCUGAUUUGCACCGCUAA GAGAAUGAGAGUGAAAGCCGCCAGAGUGGAUAAGUACCCUUACGACGUGCCCGA UUAUGCUGGAAGCUCUGGAGUGUCUGGCUGGAGGCUGUUCAAGAAGAUCAGC |
| 22 | VZVE-3-2 mRNA sequence | AUGGGCACCGUGAACAAGCCUGUGGUGGGCGUGCUCAUGGGCUUUGGCAUCAUC ACAGGCACCCUGAGAAUCACCAAUCCCGUGAGAGCCAGCGUGCUGCGGUACGACG ACUUCCACAUCGACGAGGACAAGCUGGACACCAAUAGCGUGUACGAGCCCUACU ACCACAGCGACCACGCCGAGAGCAGCUGGGUGAAUAGAGGAGAAUCUAGCAGGA AGGCCUAUGACCACAAUAGCCCAUACAUUUGGCCCAGGAAUGAUUACGACGGCU UCCUGGAGAACGCCCACGAGCACCACGGCGUGUACAACCAGGGAAGAGGCAUUG AUUCUGGCGAACGGCUGAUGCAGCCAACACAGAUGAGCGCCCAGGAGGACCUGG GAGAUGAUACCGGAAUCCACGUGAUUCCUACCCUGAACGGCGAUGAUAGACACA AGAUCGUGAACGUGGACCAGAGACAGUACGGCGACGUGUUUAAGGGCGACCUGA ACCCCAAGCCUCAGGGACAGAGGCUGAUUGAGGUGAGCGUGGAAGAGAACCACC CUUUCACACUGAGAGCCCCCAUCCAGAGGAUUUACGGCGUGAGGUACACAGAGA CAUGGAGCUUUCUGCCUAGCCUGACAUGUACAGGCGAUGCCGCCCCUGCCAUUCA GCACAUCUGCCUGAAGCACACCACCUGUUUUCAGGAUGUGGUGGUGGAUGUGGA UUGCGCCGAGAACACCAAGGAGGAUCAGCUGGCCGAGAUCAGCUACAGAUUCCA GGGAAAGAAGGAGGCCGACCAGCCUUGGAUCGUGGUGAAUACAAGCACCCUGUU CGAUGAGCUGGAGCUGGACCCUCCCGAGAUUGAACCUGGCGUGCUGAAGGUGCU GAGGACCGAGAAGCAGUAUCUGGGAGUGUACAUUUGGAAUAUGAGGGGCAGCGA CGGCACAUCUACCUACGCCACCUUCCUGGUGACAUGGAAGGGCGACGAAAAAACC CGGAAUCCUACCCCUGCCGUGACCCCACAGCCUAGAGGAGCCGAGUUUCACAUGU GGAAUUACCACAGCCACGUGUUCAGCGUGGGAGACACAUUCUCUCUGGCCAUGC ACCUGCAGUACAAGAUUCACGAAGCCCCAUUCGACCUGCUGCUGGAGUGGCUGU AUGUGCCCAUUGAUCCCACCUGUCAGCCCAUGCGACUGUACAGCACCUGUCUGUA UCACCCAAACGCCCCCCAGUGUCUGUCUCACAUGAAUAGCGGAUGCACCUUUACA AGCCCACAUCUGGCCCAGAGGGUGGCUAGCACAGUGUACCAGAAUUGCGAGCAC GCCGACAACUACACAGCUUACUGCCUGGGCAUCAGCCACAUGGAGCCUUCUUUCG GCCUGAUCCUGCACGACGGCGGCACAACACUGAAAUUUGUGGAUACCCCAGAGA GCCUGUCUGGCCUGUACGUGUUUGUGGUGUAUUUUAACGGCCACGUGGAGGCCG UGGCCUACACAGUGGUGUCUACCGUGGACCACUUUGUGAACGCUAUCGAGGAGA GAGGCUUUCCUCCAACAGCUGGACAGCCUCCAGCUACCACAAAGCCUAAGGAGAU CACCCCUGUGAACCCCGGAACAUCUCCACUGCUGAGAUACGCCGCUUGGACCGGC GGAUUGGCUGCCGUUGUGCUGCUGUGCCUGGUGAUCUUUCUGAUUUGUACAGCC AAGAGAAUGAGAGUGAAGGCUGCUCGGGUGGACAAGUAUCCAUACGAUGUGCCC GAUUAUGCCGGAUCUUCUGGCGUGAGCGGCUGGAGAUUGUUCAAGAAGAUCUCC |
| 23 | VZVE-3-3 mRNA sequence | AUGGGCACCGUGAACAAGCCUGUGGUGGGAGUGCUCAUGGGAUUUGGAAUCAUU ACAGGCACACUGAGGAUCACCAACCCCGUGAGAGCCAGCGUGCUGAGGUACGAU GACUUCCACAUCGACGAGGACAAGCUGGACACCAACAGCGUGUACGAGCCCUACU ACCACAGCGACCACGCCGAGAGCUCUUGGGUGAAUAGGGGCGAAUCUAGCAGAA AGGCCUACGACCACAAUAGCCCCUAUAUUUGGCCUAGAAACGACUACGAUGGCU UCCUGGAGAACGCCCACGAGCACCACGGAGUGUACAAUCAGGGCAGAGGCAUCG AUAGCGGCGAAAGGCUGAUGCAGCCUACCCAGAUGAGCGCCCAGGAAGAUCUGG GCGAUGACACAGGCAUCCAUGUGAUUCCUACACUGAACGGCGACGACAGGCACA AAAUCGUGAAUGUGGAUCAGAGGCAGUACGGCGACGUGUUUAAGGGCGAUCUGA AUCCCAAGCCACAGGGCCAGAGGCUGAUCGAAGUGAGCGUGGAGGAGAAUCACC CUUUUACCCUGCGGGCCCCAAUCCAGCGGAUCUACGGAGUGAGGUAUACCGAAA CAUGGAGCUUUCUGCCAAGCCUGACCUGUACCGGAGACGCUGCCCCUGCUAUCCA GCACAUUUGUCUGAAGCACACCACCUGUUUUCAGGACGUGGUGGUGGAUGUGGA UUGUGCCGAGAACACCAAGGAGGACCAGCUGGCCGAGAUCAGCUACAGAUUCCA GGGCAAGAAGGAGGCCGAUCAGCCAUGGAUUGUUGUGAAUACCAGCACCCUGUU CGACGAGCUGGAGCUGGACCCUCCAGAAAUUGAGCCUGGCGUGCUGAAGGUGCU GAGAACCGAGAAACAGUACCUGGGAGUUUACAUCUGGAACAUGAGGGGCUCCGA CGGCACAAGCACCUACGCUACCUUCCUGGUGACAUGGAAGGGCGACGAGAAGAC AAGAAAUCCUACCCCAGCCGUGACACCACAGCCCAGGGGCGCCGAGUUUCAUAUG UGGAAUUACCACAGCCACGUGUUCAGCGUGGGCGAUACAUUUAGCCUGGCCAUG CAUCUGCAGUACAAGAUCCACGAGGCUCCUUUCGACCUGCUGCUGGAAUGGCUG UACGUGCCAAUCGACCCCACAUGCCAGCCUAUGAGGCUGUAUAGCACAUGCCUGU AUCACCCUAACGCCCCCCAGUGCCUGAGCCACAUGAACAGCGGAUGCACAUUUAC CUCUCCUCACCUGGCCCAGAGAGUGGCCAGCACAGUGUAUCAGAAUUGCGAGCAC GCCGACAAUUAUACUGCCUACUGCCUGGGCAUUAGCCACAUGGAGCCCAGCUUU GGCCUGAUCCUGCAUGAUGGCGGCACAACACUGAAAUUCGUGGAUACCCCCGAG AGCCUGUCUGGCCUGUACGUGUUCGUGGUGUACUUUAAUGGCCACGUGGAGGCC GUGGCCUACACAGUGGUUAGCACAGUGGAUCACUUCGUGAACGCCAUCGAAGAG AGAGGCUUUCCACCUACAGCCGGACAGCCUCCUGCUACCACAAAACCCAAGGAGA UCACCCCUGUGAAUCCUGGAACCAGCCCACUGCUGAGAUAUGCUGCUUGGACAG GCGGCCUGGCUGCUGUGGUGCUGCUGUGUCUGGUGAUCUUUCUGAUCUGUACCG CUAAGAGGAUGAGGGUGAAGGCCGCCAGAGUGGAUAAAUAUCCCUACGAUGUGC CUGAUUAUGCCGGAAGCUCUGGCGUGUCUGGAUGGAGGCUGUUCAAGAAGAUCA GC |
| 24 | VZVE-4-1 mRNA sequence | AUGGGCACCGUGAACAAGCCUGUGGUGGGCGUGCUGAUGGGAUUUGGAAUCAUC ACCGGCACCCUGAGAAUCACCAACCCUGUGAGAGCCAGCGUGCUGAGGUAUGAC GACUUCCAUAUCGACGAGGACAAGCUGGACACCAAUAGCGUGUACGAGCCCUAC UACCACAGCGACCACGCCGAGAGCUCUUGGGUGAAUAGAGGAGAGAGCAGCAGG AAGGCCUACGAUCACAACAGCCCUUACAUCUGGCCCAGAAACGACUAUGACGGCU UCCUGGAAAACGCCCACGAGCACCAUGGCGUGUAUAAUCAGGGCAGAGGAAUCG AUAGCGGAGAGAGGCUGAUGCAACCUACCCAGAUGUCUGCCCAGGAGGAUCUGG GCGAUGAUACCGGAAUCCACGUGAUCCCUACCCUGAACGGCGACGACAGACACAA GAUCGUGAAUGUGGACCAGAGACAGUACGGCGAUGUGUUCAAGGGCGAUCUGAA CCCCAAACCUCAGGGACAGAGGCUCAUCGAGGUGAGCGUGGAGGAGAACCAUCC UUUUACCCUGAGGGCCCCUAUCCAGAGAAUCUACGGCGUGAGGUAUACCGAGAC AUGGUCCUUCCUGCCCAGCCUGACCUGUACAGGCGAUGCCGCUCCUGCCAUCCAG CAUAUUUGCCUGAAGCAUACCACCUGUUUUCAGGAUGUGGUGGUGGAUGUGGAU UGCGCCGAGAACACCAAGGAGGACCAGCUGGCCGAGAUCUCUUAUAGAUUCCAG GGCAAGAAGGAGGCCGACCAGCCUUGGAUUGUGGUGAACACCUCUACCCUGUUC GACGAGCUGGAGCUGGACCCCCCUGAGAUUGAACCAGGCGUUCUGAAGGUGCUG AGAACCGAGAAGCAGUACCUGGGAGUGUACAUCUGGAAUAUGAGAGGCAGCGAU GGCACCAGCACCUACGCUACCUUUCUGGUGACCUGGAAGGGAGACGAGAAAACC AGGAAUCCAACCCCUGCCGUGACACCACAGCCCAGAGGCGCCGAGUUUCACAUGU GGAAUUACCACAGCCACGUGUUCAGCGUGGGCGACACCUUCUCUCUGGCCAUGCA CCUGCAGUAUAAGAUUCACGAAGCUCCAUUUGACCUGCUGCUGGAGUGGCUGUA CGUGCCAAUUGACCCCACAUGCCAGCCAAUGAGGCUGUACAGCACCUGCCUGUAC CAUCCAAACGCCCCCCAGUGCCUGUCUCACAUGAACAGCGGCUGUACAUUUACAA GCCCUCACCUGGCCCAGCGAGUGGCCAGCACAGUGUACCAGAAUUGUGAGCAUGC UGACAAUUACACAGCCUAUUGCCUGGGCAUCAGCCACAUGGAGCCAUCUUUCGG CCUGAUCCUGCACGAUGGCGGCACCACCCUGAAAUUUGUGGAUACCCCUGAGAGC CUGAGCGGCCUGUACGUGUUUGUGGUGUACUUUAACGGCCACGUGGAGGCCGUG GCCUACACCGUGGUGAGCACAGUGGAUCAUUUUGUGAAUGCCAUCGAAGAAAGA GGCUUCCCUCCUACAGCCGGACAGCCUCCUGCCACAACAAAGCCCAAGGAGAUUA CCCCAGUGAAUCCAGGAACCUCUCCACUGCUGAGAUACGCCGCUUGGACAGGAGG CCUGGCUGCUGUGGUGCUGCUGUGCCUGGUGAUCUUCCUGAUUUGCACCGCUAA GAGAAUGAGAGUGAAAGCCGCCAGAGUGGAUAAGAGCCCCUACAACCAGAGCAU GUACggcGCCGGCCUGCCUGUGGAUGAUUUCGAAGAUGCCGAAGCUGCCGAUGCU GAGGAGGAGUUUGGCAAUGCCAUUGGCGGCAGCCACGGCGGCAGCAGCUAUACC GUGUACAUCGACAAGACAAGGUACCCUUACGACGUGCCCGAUUAUGCUGGAAGC UCUGGAGUGUCUGGCUGGAGGCUGUUCAAGAAGAUCAGC |
| 25 | VZVE-4-2 mRNA sequence | AUGGGCACCGUGAACAAGCCUGUGGUGGGCGUGCUCAUGGGCUUUGGCAUCAUC ACAGGCACCCUGAGAAUCACCAAUCCCGUGAGAGCCAGCGUGCUGCGGUACGACG ACUUCCACAUCGACGAGGACAAGCUGGACACCAAUAGCGUGUACGAGCCCUACU ACCACAGCGACCACGCCGAGAGCAGCUGGGUGAAUAGAGGAGAAUCUAGCAGGA AGGCCUAUGACCACAAUAGCCCAUACAUUUGGCCCAGGAAUGAUUACGACGGCU UCCUGGAGAACGCCCACGAGCACCACGGCGUGUACAACCAGGGAAGAGGCAUUG AUUCUGGCGAACGGCUGAUGCAGCCAACACAGAUGAGCGCCCAGGAGGACCUGG GAGAUGAUACCGGAAUCCACGUGAUUCCUACCCUGAACGGCGAUGAUAGACACA AGAUCGUGAACGUGGACCAGAGACAGUACGGCGACGUGUUUAAGGGCGACCUGA ACCCCAAGCCUCAGGGACAGAGGCUGAUUGAGGUGAGCGUGGAAGAGAACCACC CUUUCACACUGAGAGCCCCCAUCCAGAGGAUUUACGGCGUGAGGUACACAGAGA CAUGGAGCUUUCUGCCUAGCCUGACAUGUACAGGCGAUGCCGCCCCUGCCAUUCA GCACAUCUGCCUGAAGCACACCACCUGUUUUCAGGAUGUGGUGGAUGUGGA UUGCGCCGAGAACACCAAGGAGGAUCAGCUGGCCGAGAUCAGCUACAGAUUCCA GGGAAAGAAGGAGGCCGACCAGCCUUGGAUCGUGGUGAAUACAAGCACCCUGUU CGAUGAGCUGGAGCUGGACCCUCCCGAGAUUGAACCUGGCGUGCUGAAGGUGCU GAGGACCGAGAAGCAGUAUCUGGGAGUGUACAUUUGGAAUAUGAGGGGCAGCGA CGGCACAUCUACCUACGCCACCUUCCUGGUGACAUGGAAGGGCGACGAAAAAACC CGGAAUCCUACCCCUGCCGUGACCCCACAGCCUAGAGGAGCCGAGUUUCACAUGU GGAAUUACCACAGCCACGUGUUCAGCGUGGGAGACACAUUCUCUCUGGCCAUGC ACCUGCAGUACAAGAUUCACGAAGCCCCAUUCGACCUGCUGCUGGAGUGGCUGU AUGUGCCCAUUGAUCCCACCUGUCAGCCCAUGCGACUGUACAGCACCUGUCUGUA UCACCCAAACGCCCCCCAGUGUCUGUCUCACAUGAAUAGCGGAUGCACCUUUACA AGCCCACAUCUGGCCCAGAGGGUGGCUAGCACAGUGUACCAGAAUUGCGAGCAC GCCGACAACUACACAGCUUACUGCCUGGGCAUCAGCCACAUGGAGCCUUCUUUCG GCCUGAUCCUGCACGACGGCGGCACAACACUGAAAUUUGUGGAUACCCCAGAGA GCCUGUCUGGCCUGUACGUGUUUGUGGUGUAUUUUAACGGCCACGUGGAGGCCG UGGCCUACACAGUGGUGUCUACCGUGGACCACUUUGUGAACGCUAUCGAGGAGA GAGGCUUUCCUCCAACAGCUGGACAGCCUCCAGCUACCACAAAGCCUAAGGAGAU CACCCCUGUGAACCCCGGAACAUCUCCACUGCUGAGAUACGCCGCUUGGACCGGC GGAUUGGCUGCCGUUGUGCUGCUGUGCCUGGUGAUCUUUCUGAUUUGUACAGCC AAGAGAAUGAGAGUGAAGGCUGCUCGGGUGGACAAGAGCCCUUACAAUCAGAGC AUGUACggcGCCGGCCUGCCUGUGGAUGACUUCGAAGAUGCCGAAGCUGCCGAUG CCGAGGAGGAAUUUGGCAAUGCUAUCGGCGGCAGCCAUGGCGGCAGCAGCUACA CCGUGUACAUCGACAAAACCAGGUAUCCAUACGAUGUGCCCGAUUAUGCCGGAU CUUCUGGCGUGAGCGGCUGGAGAUUGUUCAAGAAGAUCUCC |
| 26 | VZVE-4-3 mRNA sequence | AUGGGCACCGUGAACAAGCCUGUGGUGGGAGUGCUCAUGGGAUUUGGAAUCAUU ACAGGCACACUGAGGAUCACCAACCCCGUGAGAGCCAGCGUGCUGAGGUACGAU GACUUCCACAUCGACGAGGACAAGCUGGACACCAACAGCGUGUACGAGCCCUACU ACCACAGCGACCACGCCGAGAGCUCUUGGGUGAAUAGGGGCGAAUCUAGCAGAA AGGCCUACGACCACAAUAGCCCCUAUAUUUGGCCUAGAAACGACUACGAUGGCU UCCUGGAGAACGCCCACGAGCACCACGGAGUGUACAAUCAGGGCAGAGGCAUCG AUAGCGGCGAAAGGCUGAUGCAGCCUACCCAGAUGAGCGCCCAGGAAGAUCUGG GCGAUGACACAGGCAUCCAUGUGAUUCCUACACUGAACGGCGACGACAGGCACA AAAUCGUGAAUGUGGAUCAGAGGCAGUACGGCGACGUGUUUAAGGGCGAUCUGA AUCCCAAGCCACAGGGCCAGAGGCUGAUCGAAGUGAGCGUGGAGGAGAAUCACC CUUUUACCCUGCGGGCCCCAAUCCAGCGGAUCUACGGAGUGAGGUAUACCGAAA CAUGGAGCUUUCUGCCAAGCCUGACCUGUACCGGAGACGCUGCCCCUGCUAUCCA GCACAUUUGUCUGAAGCACACCACCUGUUUUCAGGACGUGGUGGUGGAUGUGGA UUGUGCCGAGAACACCAAGGAGGACCAGCUGGCCGAGAUCAGCUACAGAUUCCA GGGCAAGAAGGAGGCCGAUCAGCCAUGGAUUGUUGUGAAUACCAGCACCCUGUU CGACGAGCUGGAGCUGGACCCUCCAGAAAUUGAGCCUGGCGUGCUGAAGGUGCU GAGAACCGAGAAACAGUACCUGGGAGUUUACAUCUGGAACAUGAGGGGCUCCGA CGGCACAAGCACCUACGCUACCUUCCUGGUGACAUGGAAGGGCGACGAGAAGAC AAGAAAUCCUACCCCAGCCGUGACACCACAGCCCAGGGGCGCCGAGUUUCAUAUG UGGAAUUACCACAGCCACGUGUUCAGCGUGGGCGAUACAUUUAGCCUGGCCAUG CAUCUGCAGUACAAGAUCCACGAGGCUCCUUUCGACCUGCUGCUGGAAUGGCUG UACGUGCCAAUCGACCCCACAUGCCAGCCUAUGAGGCUGUAUAGCACAUGCCUGU AUCACCCUAACGCCCCCCAGUGCCUGAGCCACAUGAACAGCGGAUGCACAUUUAC CUCUCCUCACCUGGCCCAGAGAGUGGCCAGCACAGUGUAUCAGAAUUGCGAGCAC GCCGACAAUUAUACUGCCUACUGCCUGGGCAUUAGCCACAUGGAGCCCAGCUUU GGCCUGAUCCUGCAUGAUGGCGGCACAACACUGAAAUUCGUGGAUACCCCCGAG AGCCUGUCUGGCCUGUACGUGUUCGUGGUGUACUUUAAUGGCCACGUGGAGGCC GUGGCCUACACAGUGGUUAGCACAGUGGAUCACUUCGUGAACGCCAUCGAAGAG AGAGGCUUUCCACCUACAGCCGGACAGCCUCCUGCUACCACAAAACCCAAGGAGA UCACCCCUGUGAAUCCUGGAACCAGCCCACUGCUGAGAUAUGCUGCUUGGACAG GCGGCCUGGCUGCUGUGGUGCUGCUGUGUCUGGUGAUCUUUCUGAUCUGUACCG CUAAGAGGAUGAGGGUGAAGGCCGCCAGAGUGGAUAAAAGCCCAUACAACCAGA GCAUGggcUACGCCGGCCUGCCUGUGGAUGAUUUUGAGGAUGCUGAAGCCGCCGA UGCCGAAGAGGAGUUUGGCAAUGCCAUUGGCGGAAGCCACGGCGGCAGCAGCUA CACCGUGUACAUCGACAAGACAAGGUAUCCCUACGAUGUGCCUGAUUAUGCCGG AAGCUCUGGCGUGUCUGGAUGGAGGCUGUUCAAGAAGAUCAGC |
| 27 | VZVE-4-deltag mRNA sequence | AUGGGCACCGUGAACAAGCCUGUGGUGGGCGUGCUGAUGGGAUUUGGAAUCAUC ACCGGCACCCUGAGAAUCACCAACCCUGUGAGAGCCAGCGUGCUGAGGUAUGAC GACUUCCAUAUCGACGAGGACAAGCUGGACACCAAUAGCGUGUACGAGCCCUAC UACCACAGCGACCACGCCGAGAGCUCUUGGGUGAAUAGAGGAGAGAGCAGCAGG AAGGCCUACGAUCACAACAGCCCUUACAUCUGGCCCAGAAACGACUAUGACGGCU UCCUGGAAAACGCCCACGAGCACCAUGGCGUGUAUAAUCAGGGCAGAGGAAUCG AUAGCGGAGAGAGGCUGAUGCAACCUACCCAGAUGUCUGCCCAGGAGGAUCUGG GCGAUGAUACCGGAAUCCACGUGAUCCCUACCCUGAACGGCGACGACAGACACAA GAUCGUGAAUGUGGACCAGAGACAGUACGGCGAUGUGUUCAAGGGCGAUCUGAA CCCCAAACCUCAGGGACAGAGGCUCAUCGAGGUGAGCGUGGAGGAGAACCAUCC UUUUACCCUGAGGGCCCCUAUCCAGAGAAUCUACGGCGUGAGGUAUACCGAGAC AUGGUCCUUCCUGCCCAGCCUGACCUGUACAGGCGAUGCCGCUCCUGCCAUCCAG CAUAUUUGCCUGAAGCAUACCACCUGUUUUCAGGAUGUGGUGGUGGAUGUGGAU UGCGCCGAGAACACCAAGGAGGACCAGCUGGCCGAGAUCUCUUAUAGAUUCCAG GGCAAGAAGGAGGCCGACCAGCCUUGGAUUGUGGUGAACACCUCUACCCUGUUC GACGAGCUGGAGCUGGACCCCCCUGAGAUUGAACCAGGCGUUCUGAAGGUGCUG AGAACCGAGAAGCAGUACCUGGGAGUGUACAUCUGGAAUAUGAGAGGCAGCGAU GGCACCAGCACCUACGCUACCUUUCUGGUGACCUGGAAGGGAGACGAGAAAACC AGGAAUCCAACCCCUGCCGUGACACCACAGCCCAGAGGCGCCGAGUUUCACAUGU GGAAUUACCACAGCCACGUGUUCAGCGUGGGCGACACCUUCUCUCUGGCCAUGCA CCUGCAGUAUAAGAUUCACGAAGCUCCAUUUGACCUGCUGCUGGAGUGGCUGUA CGUGCCAAUUGACCCCACAUGCCAGCCAAUGAGGCUGUACAGCACCUGCCUGUAC CAUCCAAACGCCCCCCAGUGCCUGUCUCACAUGAACAGCGGCUGUACAUUUACAA GCCCUCACCUGGCCCAGCGAGUGGCCAGCACAGUGUACCAGAAUUGUGAGCAUGC UGACAAUUACACAGCCUAUUGCCUGGGCAUCAGCCACAUGGAGCCAUCUUUCGG CCUGAUCCUGCACGAUGGCGGCACCACCCUGAAAUUUGUGGAUACCCCUGAGAGC CUGAGCGGCCUGUACGUGUUUGUGGUGUACUUUAACGGCCACGUGGAGGCCGUG GCCUACACCGUGGUGAGCACAGUGGAUCAUUUUGUGAAUGCCAUCGAAGAAAGA GGCUUCCCUCCUACAGCCGGACAGCCUCCUGCCACAACAAAGCCCAAGGAGAUUA CCCCAGUGAAUCCAGGAACCUCUCCACUGCUGAGAUACGCCGCUUGGACAGGAGG CCUGGCUGCUGUGGUGCUGCUGUGCCUGGUGAUCUUCCUGAUUUGCACCGCUAA GAGAAUGAGAGUGAAAGCCGCCAGAGUGGAUAAGAGCCCCUACAACCAGAGCAU GUACggcGCCGGCCUGCCUGUGGAUGAUUUCGAAGAUGCCGAAGCUGCCGAUGCU GAGGAGGAGUUUGGCAAUGCCAUUGGCGGCAGCCACGGCGGCAGCAGCUAUACC GUGUACAUCGACAAGACAAGG |
| 28 | VZVE-5-1 mRNA sequence | AUGGGCACCGUGAAUAAGCCAGUGGUGGGCGUGCUGAUGGGCUUUGGCAUUAUC ACCGGAACACUGAGGAUCACCAACCCAGUGAGGGCCUCUGUGCUGAGAUACGAC GACUUCCACAUCGACGAGGACAAGCUGGACACCAACAGCGUGUACGAGCCAUAC UACCACAGCGAUCAUGCCGAGAGCUCUUGGGUGAACAGGGGAGAGAGCAGCAGG AAGGCCUACGACCACAAUUCUCCCUAUAUCUGGCCCAGGAACGACUACGAUGGCU UCCUGGAGAAUGCCCAUGAACACCACGGAGUGUACAACCAGGGCAGAGGCAUUG ACUCUGGAGAGAGGCUGAUGCAGCCAACCCAGAUGUCUGCCCAGGAGGAUCUGG GAGACGAUACCGGCAUCCAUGUGAUUCCCACACUGAACGGAGACGACAGGCACA AGAUCGUGAAUGUGGACCAGAGACAGUACGGCGAUGUGUUCAAGGGCGACCUGA AUCCUAAGCCUCAGGGCCAGCGACUGAUUGAGGUGUCUGUGGAGGAGAACCAUC CUUUCACACUGCGGGCUCCUAUCCAGCGGAUCUACGGCGUGAGAUACACCGAAAC CUGGAGCUUUCUGCCAAGCCUGACCUGUACAGGCGAUGCCGCUCCUGCUAUCCAG CACAUCUGCCUGAAGCAUACAACAUGCUUUCAGGACGUGGUGGUGGAUGUGGAU UGCGCCGAGAACACCAAAGAGGACCAGCUGGCCGAGAUUAGCUACAGAUUCCAG GGCAAGAAGGAGGCCGAUCAGCCCUGGAUCGUGGUGAAUACCUCUACCCUGUUU GACGAGCUGGAGCUGGACCCCCCUGAGAUUGAGCCUGGAGUGCUGAAGGUGCUG AGAACAGAGAAGCAGUACCUGGGCGUGUACAUUUGGAACAUGAGAGGCAGCGAC GGCACCUCUACCUAUGCCACAUUUCUGGUGACCUGGAAGGGCGACGAGAAAACC AGAAAUCCAACACCUGCCGUGACACCCCAGCCCAGGGGAGCCGAGUUUCACAUGU GGAAUUACCACAGCCACGUGUUCAGCGUGGGCGACACAUUUAGCCUGGCCAUGC ACCUGCAGUACAAAAUCCACGAGGCUCCUUUCGAUCUGCUGCUGGAGUGGCUGU AUGUGCCUAUUGACCCUACCUGCCAGCCUAUGAGGCUGUACAGCACCUGUCUGU ACCACCCAAACGCCCCUCAGUGCCUGAGCCAUAUGAACAGCGGCUGUACAUUCAC CUCUCCACACCUGGCCCAGAGAGUGGCCAGCACCGUGUAUCAGAAUUGCGAGCAU GCCGAUAACUACACAGCCUACUGUCUGGGCAUUAGCCACAUGGAGCCUAGCUUU GGCCUGAUCCUGCACGAUGGAGGCACCACCCUGAAAUUCGUGGAUACCCCCGAGA GCCUGAGCGGCCUGUACGUGUUUGUGGUGUACUUCAAUGGACACGUGGAGGCCG UGGCCUACACAGUGGUGAGCACAGUGGAUCACUUCGUGAACGCUAUUGAGGAGA GAGGAUUUCCUCCAACAGCCGGCCAGCCUCCAGCCACAACCAAACCCAAAGAAAU CACCCCCGUGAAUCCUGGAACAUCUCCCCUGCUGAGGUAUGCUGCUUGGACAGGA GGCCUGGCUGCCGUGGUGCUGCUGUGCCUGGUGAUCUUUCUGAUCUGUACCGCU AAGAGAAUGAGGGUGAAGGCCUAUCCAUACGAUGUGCCCGAUUAUGCCGGAUCU UCUGGAGUGUCUGGCUGGCGGCUGUUCAAGAAGAUCAGC |
| 29 | VZVE-5-2 mRNA sequence | AUGGGCACCGUGAACAAGCCUGUGGUGGGAGUGCUGAUGGGCUUCGGAAUCAUU ACAGGCACACUGAGAAUCACCAAUCCUGUGAGAGCCAGCGUGCUGCGGUACGAC GACUUCCACAUCGACGAGGACAAGCUGGACACCAACAGCGUGUACGAGCCCUACU ACCACAGCGACCACGCCGAGAGCAGCUGGGUGAAUAGGGGCGAAUCUAGCAGAA AAGCCUACGACCACAAUAGCCCUUACAUCUGGCCUAGGAACGAUUACGACGGAU UUCUGGAGAAUGCUCACGAGCACCACGGCGUGUACAAUCAGGGCAGGGGCAUUG ACUCUGGCGAGAGGCUGAUGCAGCCUACCCAGAUGAGCGCCCAGGAAGAUCUGG GCGACGACACAGGCAUCCAUGUGAUCCCUACACUGAAUGGAGACGACAGACACA AGAUCGUGAAUGUGGAUCAGAGACAGUACGGCGACGUGUUUAAAGGCGAUCUGA ACCCCAAGCCUCAGGGCCAGCGGCUGAUCGAGGUGUCUGUGGAGGAGAACCACCC UUUUACACUGCGGGCCCCUAUCCAGCGGAUCUACGGCGUGAGAUACACAGAGAC AUGGAGCUUCCUGCCUUCUCUGACCUGUACAGGAGACGCUGCUCCUGCCAUUCAG CACAUCUGCCUGAAACACACCACAUGCUUUCAGGACGUGGUGGUGGACGUGGAU UGUGCCGAGAAUACCAAGGAGGACCAGCUGGCCGAGAUCAGCUACAGGUUCCAG GGAAAGAAGGAGGCCGACCAGCCUUGGAUUGUGGUGAACACCUCUACCCUGUUU GAUGAGCUGGAGCUGGACCCUCCAGAGAUUGAGCCCGGCGUGCUGAAAGUGCUG AGAACCGAGAAGCAGUAUCUGGGCGUGUAUAUCUGGAACAUGAGGGGCAGCGAU GGAACCAGCACCUACGCCACCUUCCUGGUGACAUGGAAGGGCGACGAGAAGACA AGGAAUCCUACACCAGCCGUGACACCUCAGCCAAGAGGCGCCGAGUUUCACAUGU GGAAUUAUCACUCUCACGUGUUCAGCGUGGGCGACACCUUCAGCCUGGCCAUGC AUCUGCAGUAUAAGAUCCACGAGGCCCCCUUUGAUCUGCUGCUGGAGUGGCUGU AUGUGCCUAUCGACCCAACCUGCCAGCCAAUGAGGCUGUACUCUACCUGCCUGUA CCACCCAAACGCCCCUCAGUGCCUGAGCCACAUGAACAGCGGCUGUACAUUUACA AGCCCCCAUCUUGCUCAGAGGGUGGCCUCUACAGUGUACCAGAAUUGCGAGCAC GCCGAUAAUUACACAGCCUACUGCCUGGGCAUCAGCCACAUGGAGCCUAGCUUU GGCCUGAUCCUGCACGAUGGCGGCACCACCCUGAAGUUUGUGGAUACACCUGAG AGCCUGAGCGGCCUGUACGUGUUCGUGGUGUACUUUAACGGACAUGUGGAGGCC GUGGCCUAUACAGUGGUGAGCACCGUGGACCAUUUUGUGAAUGCCAUUGAGGAG AGAGGAUUCCCUCCUACAGCCGGACAGCCUCCAGCCACCACCAAGCCAAAGGAAA UCACACCCGUGAAUCCAGGAACCUCUCCUCUGCUGAGAUAUGCUGCCUGGACAGG CGGCCUGGCUGCUGUGGUGCUGCUGUGUCUGGUUAUCUUCCUGAUCUGUACAGC UAAGAGGAUGCGGGUGAAGGCCUACCCUUACGAUGUGCCAGAUUACGCCGGAAG CUCUGGCGUGUCUGGCUGGCGGCUGUUCAAGAAGAUCAGC |
| 30 | VZVE-5-3 mRNA sequence | AUGGGCACCGUGAAUAAGCCCGUGGUGGGCGUGCUGAUGGGCUUUGGCAUCAUC ACAGGAACACUGAGAAUCACCAACCCUGUGAGAGCCAGCGUGCUGAGAUACGAC GACUUUCACAUCGACGAGGACAAGCUGGACACCAACAGCGUGUACGAGCCUUAC UACCACAGCGACCACGCCGAGAGCAGCUGGGUGAAUAGGGGAGAGAGCAGCAGG AAGGCCUACGACCACAAUAGCCCAUACAUCUGGCCCAGAAACGAUUACGAUGGC UUUCUGGAAAACGCCCACGAGCAUCACGGAGUGUACAAUCAGGGAAGGGGCAUU GAUAGCGGCGAAAGGCUGAUGCAGCCCACCCAGAUGUCUGCCCAGGAGGAUCUG GGAGAUGAUACCGGCAUUCACGUGAUCCCCACCCUGAAUGGCGACGACAGGCAC AAGAUCGUGAACGUGGACCAGAGACAGUAUGGCGACGUGUUCAAAGGAGAUCUG AACCCUAAGCCUCAGGGACAGAGGCUGAUUGAGGUGAGCGUGGAGGAGAACCAC CCUUUUACCCUGCGGGCCCCUAUCCAGAGAAUCUACGGCGUGAGAUACACCGAAA CAUGGAGCUUCCUGCCUUCUCUGACAUGCACAGGCGAUGCCGCCCCUGCCAUCCA GCACAUCUGCCUGAAGCACACAACAUGCUUUCAGGAUGUGGUGGUGGAUGUGGA UUGUGCCGAGAACACAAAGGAGGACCAGCUGGCCGAGAUCUCUUACAGAUUCCA GGGCAAGAAGGAGGCCGACCAGCCUUGGAUCGUGGUGAAUACCUCUACCCUGUU CGAUGAGCUGGAGCUGGACCCACCAGAGAUCGAGCCUGGCGUGCUCAAGGUGCU GAGAACCGAGAAACAGUACCUGGGCGUGUACAUUUGGAACAUGAGAGGCUCUGA CGGCACAUCUACCUACGCCACCUUUCUGGUGACCUGGAAGGGAGACGAGAAGAC CAGAAACCCAACACCUGCCGUGACACCCCAGCCAAGAGGCGCCGAGUUUCACAUG UGGAAUUAUCACAGCCACGUGUUCAGCGUGGGCGACACCUUCAGCCUGGCUAUG CAUCUGCAGUAUAAGAUCCACGAAGCCCCAUUCGACCUGCUGCUGGAGUGGCUG UAUGUGCCAAUCGACCCAACAUGCCAGCCCAUGCGCCUGUACAGCACCUGUCUGU AUCAUCCUAACGCCCCACAGUGCUUAAGCCACAUGAACAGCGGCUGUACAUUCAC AUCUCCACACCUCGCCCAGAGGGUGGCCAGCACCGUGUACCAGAAUUGUGAACAU GCCGACAAUUACACCGCCUACUGCCUGGGAAUCAGCCACAUGGAGCCUAGCUUCG GCCUGAUCCUGCACGAUGGAGGCACAACCCUGAAGUUUGUGGACACACCCGAGU CACUGAGCGGCCUGUACGUGUUUGUGGUGUACUUCAAUGGACACGUGGAGGCCG UGGCCUACACAGUGGUGUCUACCGUGGAUCAUUUCGUGAACGCUAUCGAGGAGA GAGGAUUUCCUCCUACAGCUGGACAGCCUCCCGCCACCACCAAACCCAAGGAGAU UACACCUGUGAAUCCAGGCACAUCUCCCCUGCUGCGGUAUGCUGCUUGGACAGG AGGCCUGGCUGCUGUGGUGCUGCUGUGUCUGGUGAUCUUCCUGAUCUGCACCGC UAAAAGAAUGAGGGUGAAAGCCUACCCUUACGAUGUCCCUGACUAUGCCGGCUC UAGCGGAGUGUCUGGCUGGAGAUUGUUCAAGAAGAUCAGC |
| 31 | VZVE-6-1 mRNA sequence | AUGGGCACCGUGAACAAGCCUGUGGUGGGCGUGCUGAUGGGCUUUGGCAUCAUC ACAGGCACACUGAGGAUCACCAAUCCCGUGAGAGCCUCUGUGCUGAGAUACGAC GACUUCCACAUCGACGAGGACAAGCUGGACACCAACUCUGUGUACGAGCCCUACU ACCACAGCGAUCACGCCGAAAGCAGCUGGGUGAAUAGAGGCGAGAGCAGCAGAA AGGCCUACGAUCACAACAGCCCCUACAUCUGGCCUAGGAACGACUACGACGGAUU UCUGGAGAACGCCCACGAGCAUCACGGAGUGUACAAUCAGGGCAGAGGCAUUGA UAGCGGCGAGAGGCUGAUGCAGCCUACACAGAUGUCUGCUCAGGAGGAUCUGGG AGACGAUACCGGCAUUCACGUGAUCCCUACCCUGAACGGCGACGACAGACACAAG AUCGUGAACGUGGACCAGAGACAGUACGGCGACGUGUUUAAGGGCGAUCUGAAU CCUAAACCUCAGGGCCAGCGGCUGAUCGAAGUGAGCGUGGAGGAGAAUCACCCU UUCACCCUGAGAGCCCCCAUCCAGAGAAUCUACGGCGUGAGAUACACCGAAACCU GGAGCUUUCUGCCCAGCCUGACAUGUACCGGAGAUGCCGCUCCUGCCAUCCAGCA CAUCUGUCUGAAGCACACAACCUGCUUUCAGGACGUGGUGGUGGAUGUGGAUUG UGCCGAGAAUACCAAGGAGGACCAGCUGGCCGAGAUCAGCUACAGAUUCCAGGG CAAGAAAGAGGCCGAUCAGCCUUGGAUCGUGGUGAAUACCAGCACCCUGUUCGA UGAGCUGGAGCUGGACCCUCCUGAGAUCGAACCAGGAGUGCUGAAGGUGCUGAG GACCGAGAAACAGUACCUGGGCGUGUACAUCUGGAACAUGAGAGGCUCUGAUGG CACCUCUACCUACGCCACAUUCCUGGUGACCUGGAAGGGCGAUGAGAAGACAAG GAACCCAACACCUGCCGUGACACCACAGCCAAGGGGCGCCGAGUUUCACAUGUGG AAUUACCACUCUCACGUGUUCAGCGUGGGCGACACAUUCAGCCUGGCCAUGCACC UGCAGUAUAAGAUUCACGAGGCUCCUUUCGAUCUGCUGCUGGAGUGGCUGUACG UGCCUAUCGAUCCUACCUGCCAGCCAAUGAGGCUGUACAGCACCUGCCUGUACCA CCCAAAUGCUCCCCAGUGCCUGAGCCACAUGAAUAGCGGCUGUACAUUCACAAGC CCACACCUGGCCCAGAGAGUGGCCUCUACCGUGUACCAGAAUUGUGAGCAUGCCG ACAAUUACACAGCCUACUGUCUGGGCAUCAGCCACAUGGAGCCAUCCUUCGGCCU GAUCCUGCAUGACGGCGGCACAACACUGAAGUUUGUGGACACCCCUGAAAGCCU GAGCGGCCUGUAUGUGUUCGUGGUGUACUUCAAUGGCCACGUGGAGGCCGUGGC CUAUACCGUGGUGUCUACCGUGGAUCACUUUGUGAACGCCAUUGAGGAAAGAGG AUUUCCUCCUACAGCCGGACAGCCUCCUGCCACCACCAAGCCUAAGGAGAUCACC CCAGUGAAUCCAGGAACCAGCCCUCUGCUGAGACAGAUCCUGGCCAUUUACAGCA CAGUGGCUUCUAGCCUGGUGCUGGUGGUGAGCCUGGGCGCCAUCUCUUUUUGGA UGUGCAGCAAUGGCAGCCUGCAGUGCAGAAUCUGCAUC |
| 32 | VZVE-6-2 mRNA sequence | AUGGGCACCGUGAACAAGCCUGUGGUGGGCGUGCUGAUGGGCUUUGGCAUCAUC ACAGGCACACUGAGGAUCACCAAUCCCGUGAGAGCCAGCGUGCUGAGGUACGAC GACUUCCACAUCGACGAGGACAAGCUGGACACAAACUCUGUGUACGAGCCCUAC UACCACAGCGAUCACGCCGAAAGCAGCUGGGUGAAUAGAGGCGAGAGCAGCAGA AAGGCCUACGAUCACAACAGCCCCUACAUCUGGCCUAGGAAUGACUACGACGGA UUCCUGGAGAACGCCCACGAGCAUCACGGAGUGUACAAUCAGGGCAGAGGCAUU GAUAGCGGCGAGAGGCUGAUGCAGCCUACACAGAUGUCUGCUCAGGAGGAUCUG GGAGACGAUACCGGCAUUCACGUGAUCCCUACCCUGAACGGCGACGACAGACACA AGAUCGUGAACGUGGACCAGAGACAGUACGGCGACGUGUUUAAGGGCGAUCUGA AUCCUAAACCUCAGGGCCAGCGGCUGAUCGAAGUGAGCGUGGAGGAGAAUCACC CUUUCACCCUGAGAGCCCCCAUCCAGAGAAUCUACGGCGUGAGAUACACCGAAAC CUGGAGCUUUCUGCCCAGCCUGACAUGUACCGGAGAUGCCGCUCCUGCCAUCCAG CACAUCUGUCUGAAGCACACAACCUGCUUUCAGGACGUGGUGGUGGAUGUGGAU UGUGCCGAGAAUACCAAGGAGGACCAGCUGGCCGAGAUCAGCUACAGAUUCCAG GGCAAGAAGGAGGCCGAUCAGCCAUGGAUUGUGGUGAAUACCAGCACCCUGUUC GAUGAGCUGGAGCUGGACCCUCCUGAGAUCGAGCCAGGAGUGCUGAAGGUGCUG AGGACCGAAAAACAGUACCUGGGCGUGUACAUCUGGAAUAUGAGGGGCUCUGAU GGCACCUCUACCUACGCCACAUUCCUGGUGACCUGGAAGGGCGAUGAGAAGACA AGGAACCCAACACCUGCCGUGACACCACAGCCAAGGGGCGCCGAGUUUCACAUGU GGAAUUACCACUCUCACGUGUUCAGCGUGGGCGACACAUUCAGCCUGGCCAUGC ACCUGCAGUAUAAGAUUCACGAGGCUCCUUUCGAUCUGCUGCUGGAGUGGCUGU ACGUGCCCAUCGAUCCUACCUGUCAGCCAAUGAGGCUGUACAGCACCUGCCUGUA CCACCCAAAUGCCCCCCAGUGCCUGAGCCAUAUGAACAGCGGCUGUACAUUUACA AGCCCACACCUGGCCCAGAGAGUGGCCUCUACCGUGUACCAGAAUUGUGAGCAU GCCGACAAUUACACAGCCUACUGUCUGGGCAUCAGCCACAUGGAGCCAUCCUUCG GCCUGAUCCUGCAUGACGGCGGCACAACACUGAAGUUUGUGGACACCCCUGAAA GCCUGAGCGGCCUGUAUGUGUUCGUGGUGUACUUCAAUGGCCACGUGGAGGCCG UGGCCUAUACCGUGGUGUCUACCGUGGAUCACUUUGUGAACGCCAUUGAGGAAA GAGGAUUUCCUCCUACAGCCGGACAGCCUCCUGCCACCACCAAGCCUAAGGAGAU CACACCCGUGAAUCCAGGAACCAGCCCUCUGCUGAGGCAGAUUCUGGCCAUUUAC AGCACAGUGGCUUCUAGCCUGGUGCUGGUGGUGAGCCUGGGCGCCAUCUCUUUU UGGAUGUGCAGCAACGGCAGCCUGCAGUGUAGAAUCUGCAUC |
| 33 | VZVE-6-3 mRNA sequence | AUGGGCACCGUGAACAAGCCUGUGGUGGGCGUGCUGAUGGGCUUUGGAAUCAUC ACAGGCACACUGAGGAUCACCAAUCCCGUGAGAGCCAGCGUGCUGAGGUACGAC GACUUCCACAUCGACGAGGACAAGCUGGACACAAACUCUGUGUACGAGCCCUAC UACCACAGCGAUCACGCCGAAAGCAGCUGGGUGAAUAGAGGCGAGAGCAGCAGA AAGGCCUACGAUCACAACAGCCCCUACAUCUGGCCUAGGAAUGACUACGACGGA UUCCUGGAAAACGCCCACGAGCACCACGGAGUGUACAAUCAGGGCAGAGGCAUU GAUAGCGGCGAGAGGCUGAUGCAGCCUACACAGAUGUCUGCUCAGGAAGAUCUG GGAGACGACACCGGCAUUCACGUGAUCCCUACCCUGAACGGCGACGAUAGACACA AGAUCGUGAACGUGGACCAGAGGCAGUAUGGCGACGUGUUUAAGGGCGACCUGA AUCCUAAGCCUCAGGGACAGCGGCUGAUUGAAGUGAGCGUGGAGGAGAACCACC CAUUCACCCUGAGAGCCCCAAUCCAGAGAAUCUACGGCGUGAGAUACACCGAAAC CUGGAGCUUCCUGCCCAGCCUGACAUGCACCGGAGAUGCAGCUCCUGCUAUCCAG CACAUCUGCCUGAAGCACACAACCUGUUUCCAGGAUGUUGUGGUUGAUGUGGAU UGCGCCGAGAACACCAAGGAGGACCAGCUGGCCGAGAUCAGCUACAGAUUCCAG GGCAAGAAGGAGGCCGAUCAGCCAUGGAUUGUGGUGAAUACCAGCACCCUGUUC GAUGAGCUGGAGCUGGACCCUCCUGAGAUCGAGCCAGGAGUGCUGAAGGUGCUG AGGACCGAAAAGCAGUACCUGGGCGUGUAUAUCUGGAAUAUGAGGGGCUCUGAU GGCACCUCUACCUACGCCACAUUCCUGGUGACCUGGAAGGGCGAUGAGAAAACA AGGAACCCUACACCUGCCGUGACACCACAGCCCAGGGGCGCCGAGUUUCACAUGU GGAAUUACCACUCUCACGUGUUCAGCGUGGGCGACACAUUUAGCCUGGCCAUGC ACCUGCAGUACAAAAUUCACGAGGCUCCCUUCGAUCUGCUGCUGGAGUGGCUGU AUGUGCCCAUCGAUCCCACCUGUCAGCCAAUGAGGCUGUAUAGCACAUGCCUGU ACCACCCAAACGCCCCCCAGUGCCUGAGCCAUAUGAAUAGCGGCUGCACAUUUAC AAGCCCACACCUGGCCCAGAGAGUGGCCAGUACCGUGUACCAGAAUUGUGAGCA UGCCGAUAACUACACAGCCUACUGUCUGGGCAUCAGCCACAUGGAGCCAUCCUUC GGCCUGAUCCUGCACGACGGCGGCACAACACUGAAGUUUGUGGACACACCCGAG AGCCUGAGCGGCCUGUAUGUGUUUGUGGUGUACUUUAAUGGCCACGUGGAGGCU GUGGCCUACACCGUGGUGUCUACAGUGGAUCACUUUGUGAAUGCCAUCGAGGAA AGAGGAUUCCCUCCUACAGCCGGACAGCCUCCUGCCACCACCAAACCCAAGGAGA UCACACCCGUGAAUCCAGGCACCAGCCCUCUGCUGAGGCAGAUUCUGGCCAUUUA UAGUACAGUGGCUUCUAGCCUGGUGCUGGUGGUGAGCCUGGGCGCCAUCUCUUU UUGGAUGUGUAGCAACGGCAGCCUGCAGUGUAGAAUCUGCAUC |
| 34 | VZVE-7-1 mRNA sequence | AUGGGCACCGUGAAUAAGCCCGUGGUGGGCGUGCUGAUGGGCUUUGGCAUCAUC ACAGGCACCCUGAGGAUCACCAAUCCAGUGAGAGCCUCUGUGCUGAGGUACGAC GACUUCCACAUCGAUGAGGACAAGCUGGACACCAAUAGCGUGUACGAGCCCUAC UACCACAGCGAUCAUGCCGAGAGCAGCUGGGUGAAUAGAGGAGAGAGCAGCAGA AAGGCCUACGACCACAACAGCCCCUACAUCUGGCCCAGGAACGACUACGAUGGCU UCCUGGAGAAUGCCCACGAACAUCACGGCGUGUACAACCAGGGAAGAGGCAUCG AUUCUGGAGAAAGGCUGAUGCAGCCUACCCAGAUGUCUGCCCAGGAGGAUCUGG GCGAUGAUACAGGAAUCCAUGUGAUCCCUACCCUGAACGGCGACGACAGGCACA AGAUCGUGAACGUGGACCAGAGGCAGUAUGGCGACGUGUUCAAGGGCGACCUGA AUCCUAAGCCUCAGGGACAGAGGCUGAUUGAGGUGAGCGUGGAAGAGAAUCACC CUUUCACCCUGAGAGCCCCUAUCCAGAGGAUCUACGGCGUGAGGUAUACAGAGA CAUGGAGCUUCCUGCCUUCUCUGACCUGUACCGGAGAUGCCGCCCCUGCCAUUCA GCACAUCUGCCUGAAACACACCACCUGCUUUCAGGACGUGGUGGUGGAUGUUGA CUGUGCCGAGAACACCAAGGAGGAUCAGCUGGCCGAAAUCAGCUACAGGUUCCA GGGCAAGAAAGAGGCCGAUCAGCCUUGGAUUGUGGUGAACACCAGCACCCUGUU UGAUGAGCUGGAGCUGGACCCUCCUGAGAUCGAGCCUGGAGUGCUGAAGGUGCU GAGGACCGAGAAGCAGUACCUGGGAGUGUACAUCUGGAAUAUGAGGGGCUCUGA CGGCACAAGCACAUAUGCCACCUUCCUGGUGACCUGGAAGGGCGAUGAGAAGAC CAGAAACCCUACACCUGCUGUGACACCACAGCCUAGGGGCGCCGAGUUUCACAUG UGGAAUUACCACAGCCACGUGUUCUCUGUGGGCGACACCUUCUCCCUGGCCAUGC ACCUGCAGUAUAAGAUCCAUGAGGCCCCAUUUGAUCUGCUGCUGGAGUGGCUGU ACGUGCCAAUCGAUCCAACCUGCCAGCCUAUGCGGCUGUAUAGCACCUGUCUGUA CCACCCUAACGCUCCCCAGUGCCUGAGCCACAUGAAUAGCGGAUGUACCUUUACC UCUCCUCACCUGGCCCAGAGAGUGGCCUCUACCGUGUACCAAAACUGUGAGCAUG CCGACAAUUACACCGCCUACUGUCUGGGCAUCAGCCACAUGGAGCCCUCUUUCGG CCUGAUCCUGCAUGAUGGAGGCACCACCCUGAAAUUCGUGGAUACCCCAGAGAG CCUGAGCGGCCUGUAUGUGUUUGUGGUGUACUUCAAUGGCCACGUGGAGGCCGU GGCCUAUACAGUGGUGAGCACAGUGGAUCACUUUGUGAACGCCAUCGAGGAAAG AGGCUUUCCUCCUACAGCCGGACAGCCACCUGCCACCACCAAGCCUAAGGAGAUU ACCCCAGUGAAUCCAGGAACAAGCCCACUGCUGAGGUGGCCAUGGUAUAUCUGG CUGGGAUUCAUCGCCGGCCUGAUUGCCAUCGUGAUGGUGACCAUCAUGCUGUGC UGCAUGACCAGCUGUUGUUCUUGCCUGAAGGGCUGUUGCAGCUGCGGCAGCUGC UGCAAG |
| 35 | VZVE-7-2 mRNA sequence | AUGGGCACCGUGAAUAAGCCCGUGGUGGGCGUGCUGAUGGGCUUUGGCAUUAUC ACCGGCACCCUGAGGAUCACCAAUCCAGUGAGAGCCUCUGUGCUGAGGUACGAC GACUUCCACAUCGACGAGGACAAGCUGGACACCAAUAGCGUGUACGAGCCCUAC UACCACAGCGACCAUGCCGAGUCAAGCUGGGUGAAUAGAGGAGAGAGCAGCAGA AAGGCCUACGACCACAACAGCCCAUACAUCUGGCCCAGGAACGACUACGAUGGCU UCCUGGAGAAUGCUCACGAGCAUCACGGCGUGUACAACCAGGGAAGAGGCAUCG AUUCUGGAGAAAGGCUGAUGCAGCCUACCCAGAUGUCUGCCCAGGAGGAUCUGG GCGAUGAUACAGGAAUCCAUGUGAUCCCUACCCUGAACGGCGACGACAGGCACA AGAUCGUGAACGUGGACCAGAGGCAGUACGGCGAUGUGUUUAAGGGCGACCUGA AUCCUAAGCCUCAGGGACAGAGGCUGAUCGAGGUGAGCGUGGAAGAGAAUCACC CUUUCACCCUGAGAGCCCCUAUCCAGAGGAUCUACGGCGUGAGGUAUACAGAGA CAUGGAGCUUUCUGCCUUCUCUGACCUGCACCGGAGAUGCCGCCCCUGCCAUUCA GCACAUCUGCCUGAAACACACCACCUGCUUUCAGGACGUGGUGGUGGACGUUGA UUGUGCCGAGAACACCAAGGAGGAUCAGCUGGCCGAAAUCAGCUAUAGGUUCCA GGGCAAGAAGGAGGCCGAUCAGCCCUGGAUUGUGGUGAAUACCAGCACACUGUU UGACGAGCUGGAGCUGGACCCUCCUGAGAUCGAGCCUGGAGUGCUGAAGGUGCU GAGGACCGAGAAGCAGUACCUGGGAGUGUACAUUUGGAACAUGAGGGGCUCUGA CGGCACAAGCACAUAUGCCACCUUCCUGGUGACCUGGAAGGGCGAUGAGAAGAC CAGAAACCCUACACCUGCUGUGACACCACAGCCUAGGGGCGCCGAGUUUCACAUG UGGAAUUACCACAGCCACGUGUUCUCUGUGGGCGACACCUUCUCCCUGGCCAUGC ACCUGCAGUAUAAAAUCCACGAGGCCCCAUUUGAUCUGCUGCUGGAGUGGCUGU AUGUGCCCAUCGAUCCAACCUGCCAGCCUAUGCGGCUGUAUAGCACCUGUCUGUA CCACCCUAACGCUCCCCAGUGCCUGAGCCACAUGAAUAGCGGAUGUACCUUUACC UCUCCUCACCUGGCCCAGAGAGUGGCCUCUACCGUGUACCAAAACUGUGAGCAUG CCGACAACUAUACCGCCUACUGUCUGGGCAUCAGCCACAUGGAGCCCUCUUUCGG CCUGAUCCUGCAUGAUGGAGGCACCACCCUGAAAUUCGUGGAUACCCCAGAGAG CCUGAGCGGCCUGUAUGUGUUUGUGGUGUACUUCAAUGGCCACGUGGAGGCCGU GGCCUAUACAGUGGUGAGCACAGUGGAUCACUUUGUGAACGCCAUCGAGGAAAG AGGCUUUCCUCCUACAGCCGGACAGCCACCUGCCACCACCAAGCCUAAGGAGAUU ACCCCAGUGAAUCCAGGAACAAGCCCACUGCUGAGGUGGCCAUGGUAUAUCUGG CUGGGAUUCAUCGCCGGCCUGAUUGCCAUCGUGAUGGUGACCAUCAUGCUGUGC UGCAUGACCAGCUGUUGUUCUUGCCUGAAGGGCUGUUGCAGCUGUGGCAGCUGC UGCAAG |
| 36 | VZVE-7-3 mRNA sequence | AUGGGCACCGUGAAUAAGCCCGUGGUGGGCGUGCUGAUGGGAUUUGGCAUUAUC ACCGGCACCCUGAGGAUCACCAAUCCUGUGAGAGCCUCUGUGCUGAGGUACGAC GACUUCCACAUCGACGAGGACAAGCUGGACACCAAUAGCGUGUACGAGCCCUAC UACCACUCUGACCACGCCGAGUCAAGCUGGGUGAAUAGAGGAGAGAGCAGCAGG AAGGCCUACGACCACAACUCUCCUUACAUCUGGCCCAGGAACGACUAUGACGGCU UCCUGGAGAAUGCUCACGAGCAUCACGGCGUGUACAACCAGGGACGGGGAAUCG AUUCUGGAGAAAGGCUGAUGCAGCCUACCCAGAUGUCUGCCCAGGAAGACCUGG GCGAUGAUACAGGAAUCCAUGUGAUCCCUACCCUGAACGGCGACGACAGGCACA AGAUCGUGAACGUGGACCAGAGGCAGUACGGCGAUGUGUUUAAGGGCGAUCUGA AUCCCAAACCUCAGGGCCAGAGGCUGAUCGAGGUGAGCGUGGAGGAGAAUCACC CUUUCACACUGAGAGCCCCUAUCCAGAGGAUCUACGGCGUGAGGUACACAGAAA CAUGGAGCUUUCUGCCUUCUCUGACCUGCACCGGAGAUGCCGCCCCUGCCAUUCA GCACAUCUGCCUGAAACACACCACCUGCUUUCAGGACGUGGUGGUGGACGUUGA UUGUGCCGAGAACACCAAGGAGGAUCAGCUGGCCGAAAUCAGCUAUAGGUUCCA GGGCAAGAAGGAGGCCGAUCAGCCCUGGAUUGUGGUGAAUACCAGCACACUGUU UGACGAGCUGGAGCUGGACCCUCCUGAGAUCGAGCCUGGAGUGCUGAAGGUGCU GAGGACCGAGAAGCAGUACCUGGGAGUGUACAUUUGGAACAUGAGGGGCUCUGA CGGCACAAGCACAUAUGCCACCUUCCUGGUGACCUGGAAGGGCGAUGAGAAGAC CAGAAACCCUACACCUGCUGUGACACCACAGCCUAGGGGCGCCGAGUUUCACAUG UGGAAUUACCAUAGCCACGUGUUCUCUGUGGGCGACACCUUCUCCCUGGCCAUGC ACCUGCAGUAUAAGAUCCACGAGGCCCCAUUCGAUCUGCUGCUGGAGUGGCUGU AUGUGCCAAUCGAUCCAACCUGCCAGCCUAUGCGGCUGUAUUCUACCUGUCUGU ACCAUCCCAACGCCCCCCAGUGCCUGAGCCACAUGAAUAGCGGAUGUACCUUUAC CUCUCCUCACCUGGCCCAGAGAGUGGCCUCUACCGUGUACCAAAACUGUGAGCAU GCCGACAACUAUACCGCCUAUUGCCUGGGCAUCAGCCACAUGGAACCCAGCUUCG GCCUGAUCCUGCAUGAUGGAGGCACCACCCUGAAAUUCGUGGAUACCCCAGAGA GCCUGAGCGGCCUGUACGUGUUUGUGGUGUAUUUCAAUGGCCACGUGGAGGCCG UGGCCUAUACAGUGGUGAGCACAGUGGAUCACUUUGUGAACGCCAUCGAGGAAA GAGGCUUUCCUCCUACAGCCGGACAGCCACCUGCCACCACCAAGCCUAAGGAGAU UACCCCAGUGAACCCAGGAACAAGCCCACUGCUGAGGUGGCCAUGGUAUAUCUG GCUGGGAUUCAUCGCCGGCCUGAUUGCCAUCGUGAUGGUGACCAUCAUGCUGUG CUGUAUGACCAGCUGUUGUUCUUGCCUGAAGGGCUGUUGUAGCUGUGGCAGCUG CUGCAAG |
| 37 | Hibit-HA-tag amino acid sequence | YPYDVPDYAGSSGVSGWRLFKKIS |
| 38 | VZVE-4-3 amino acid sequence | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQ PTQMSAQEDLGDDTGIHVIPTLNGDDRHKIVNVDQRQYGDVFKGDLNPKPQGQRLIEV SVEENHPFTLRAPIQRIYGVRYTETWSFLPSLTCTGDAAPAIQHICLKHTTCFQDVVVDV DCAENTKEDQLAEISYRFQGKKEADQPWIVVNTSTLFDELELDPPEIEPGVLKVLRTEK QYLGVYIWNMRGSDGTSTYATFLVTWKGDEKTRNPTPAVTPQPRGAEFHMWNYHSH VFSVGDTFSLAMHLQYKIHEAPFDLLLEWLYVPIDPTCQPMRLYSTCLYHPNAPQCLSH MNSGCTFTSPHLAQRVASTVYQNCEHADNYTAYCLGISHMEPSFGLILHDGGTTLKFV DTPESLSGLYVFVVYFNGHVEAVAYTVVSTVDHFVNAIEERGFPPTAGQPPATTKPKEI TPVNPGTSPLLRYAAWTGGLAAVVLLCLVIFLICTAKRMRVKAARVDKSPYNQSMGY AGLPVDDFEDAEAADAEEEFGNAIGGSHGGSSYTVYIDKTRYPYDVPDYAGSSGVSGW RLFKKIS |
| 39 | VZVE-4-deltag-1-1 | AUGGGCACCGUGAACAAGCCUGUGGUGGGCGUCCUGAUGGGAUUUGGAAUCAUU ACAGGCACCCUGAGGAUCACCAACCCCGUGAGAGCCAGCGUGCUGAGGUACGAU GACUUCCACAUCGACGAGGACAAGCUGGACACCAACAGCGUGUACGAGCCCUACU ACCACAGCGAUCACGCUGAGAGCUCUUGGGUGAAUAGAGGCGAGAGCAGCAGAA AGGCCUACGACCACAAUAGCCCUUACAUUUGGCCCAGAAACGACUACGACGGCUU UCUGGAGAACGCCCACGAGCACCACGGCGUGUACAAUCAGGGAAGAGGCAUCGA UUCUGGAGAGCGACUGAUGCAGCCUACCCAGAUGUCUGCCCAGGAGGAUCUGGG CGAUGAUACAGGCAUUCACGUGAUCCCUACCCUGAAUGGCGACGACAGACACAA GAUCGUGAAUGUGGACCAGAGGCAGUACGGCGAUGUGUUCAAGGGCGAUCUGAA CCCCAAGCCACAGGGACAGAGGCUGAUUGAGGUGUCUGUGGAGGAGAAUCACCC UUUCACCCUGAGAGCCCCAAUCCAGAGGAUCUACGGCGUGAGGUACACCGAAAC AUGGAGCUUCCUGCCUUCUCUGACAUGUACCGGCGAUGCCGCUCCAGCCAUUCAG CACAUCUGUCUGAAGCACACCACCUGCUUCCAGGAUGUGGUGGUGGAUGUGGAU UGCGCCGAGAAUACCAAGGAGGAUCAGCUGGCCGAGAUCAGCUACAGAUUCCAG GGCAAGAAAGAGGCCGAUCAGCCAUGGAUCGUGGUGAACACCAGCACACUGUUU GACGAGCUGGAGCUGGACCCACCUGAGAUUGAGCCAGGCGUGCUGAAGGUGCUG AGAACCGAGAAGCAGUACCUGGGAGUGUACAUCUGGAACAUGAGAGGCAGCGAU GGCACCUCUACCUACGCCACAUUUCUGGUGACCUGGAAGGGCGAUGAGAAAACC AGGAAUCCUACACCUGCCGUGACACCCCAGCCUAGAGGCGCCGAGUUUCACAUGU GGAAUUACCACAGCCACGUGUUCAGCGUGGGCGACACAUUCAGCCUGGCCAUGC ACCUGCAGUACAAGAUUCACGAAGCCCCAUUUGACCUGCUGCUGGAGUGGCUGU ACGUGCCUAUCGAUCCUACAUGCCAGCCAAUGAGGCUGUACAGCACCUGCCUGUA CCACCCAAAUGCCCCUCAGUGCCUGAGCCACAUGAAUAGCGGAUGUACCUUCACC UCUCCUCACCUGGCUCAGAGAGUGGCCAGCACAGUGUACCAGAAUUGUGAGCAC GCCGAUAACUACACCGCCUACUGCCUGGGCAUUUCUCACAUGGAGCCAUCCUUUG GCCUGAUCCUGCAUGAUGGCGGCACCACCCUGAAGUUUGUGGAUACCCCCGAGA GCCUGUCUGGCCUGUACGUGUUCGUGGUGUAUUUCAACGGCCACGUGGAAGCCG UGGCCUAUACAGUGGUGUCUACAGUGGAUCACUUUGUGAACGCCAUCGAGGAGA GAGGAUUUCCCCCUACAGCUGGACAGCCUCCUGCCACCACCAAACCUAAGGAGAU CACCCCCGUGAAUCCUGGCACAUCUCCACUGCUGAGAUACGCUGCCUGGACAGGA GGCCUGGCUGCUGUGGUGCUGCUGUGUCUGGUGAUUUUUCUGAUCUGUACCGCC AAAAGAAUGAGAGUGAAAGCCGCCAGAGUGGAUAAGAGCCCCUACAACCAGAGC AUGUAUGGCGCCGGCCUGCCUGUGGAUGAUUUUGAGGAUGCCGAGGCUGCUGAU GCCGAAGAGGAGUUUGGCAAUGCCAUUGGCGGCAGCCACGGCGGCAGCAGCUAC ACCGUGUACAUCGACAAGACCAGA |
| 40 | VZVE-4-deltag-1-2 | AUGGGCACCGUGAAUAAGCCUGUGGUGGGCGUCCUGAUGGGAUUUGGAAUCAUC ACAGGCACCCUGAGGAUCACCAACCCCGUGAGAGCCAGCGUGCUGAGGUACGAU GACUUCCACAUCGACGAGGACAAGCUGGACACCAACAGCGUGUACGAGCCCUAU UACCAUAGCGAUCACGCCGAGAGCUCUUGGGUGAAUAGAGGCGAGAGCAGCAGA AAGGCCUACGACCACAACAGCCCUUACAUCUGGCCCAGAAACGACUACGAUGGCU UUCUGGAGAACGCCCACGAGCACCACGGCGUGUACAAUCAGGGAAGAGGCAUCG AUUCUGGAGAGCGACUGAUGCAGCCAACCCAGAUGUCUGCCCAGGAGGAUCUGG GCGAUGAUACAGGAAUCCACGUGAUCCCUACCCUGAAUGGCGACGACAGACACA AGAUCGUGAAUGUGGACCAGAGGCAGUACGGCGACGUGUUUAAGGGCGAUCUGA ACCCCAAGCCACAGGGACAGAGGCUGAUUGAGGUGUCUGUGGAGGAGAAUCACC CUUUCACCCUGAGAGCCCCAAUCCAGAGGAUCUACGGCGUGAGGUACACCGAAAC AUGGAGCUUCCUGCCUUCUCUGACAUGUACCGGCGAUGCCGCUCCAGCCAUUCAG CACAUCUGUCUGAAGCACACCACCUGCUUUCAGGAUGUGGUGGUGGACGUGGAU UGCGCCGAGAAUACCAAGGAGGACCAGCUGGCCGAGAUUUCUUACAGGUUCCAG GGCAAGAAAGAGGCCGAUCAGCCAUGGAUCGUGGUGAACACCAGCACACUGUUU GACGAGCUGGAGCUGGACCCACCUGAGAUUGAGCCAGGCGUGCUGAAGGUGCUG AGAACCGAGAAGCAGUACCUGGGAGUGUACAUCUGGAACAUGAGAGGCAGCGAC GGCACCUCUACCUAUGCCACAUUUCUGGUGACCUGGAAGGGCGAUGAGAAAACC AGGAAUCCUACACCUGCCGUGACACCCCAGCCUAGAGGCGCCGAGUUUCACAUGU GGAAUUACCACAGCCACGUGUUCAGCGUGGGCGACACAUUUAGCCUGGCCAUGC ACCUGCAGUACAAGAUCCACGAGGCCCCAUUCGACCUGCUGUUAGAGUGGCUGU AUGUGCCUAUCGACCCUACAUGCCAGCCAAUGAGGCUGUACAGCACCUGUCUGU ACCACCCAAAUGCCCCUCAGUGCCUGAGCCACAUGAACAGCGGAUGUACCUUCAC CUCUCCUCACCUGGCUCAGAGAGUGGCCAGCACAGUGUACCAGAAUUGUGAGCA CGCCGACAACUACACCGCCUAUUGCCUGGGCAUCAGCCACAUGGAGCCAUCUUUU GGCUUAAUCCUGCACGAUGGCGGCACCACCCUGAAGUUUGUGGACACCCCCGAAU CUCUGUCUGGCCUGUACGUGUUCGUGGUGUAUUUCAACGGCCACGUGGAAGCCG UGGCCUACACAGUGGUGUCUACAGUGGAUCACUUCGUGAACGCCAUCGAAGAGA GAGGCUUUCCCCCUACAGCUGGACAGCCUCCUGCUACCACAAAGCCUAAGGAGAU CACCCCCGUGAAUCCUGGCACAUCUCCCCUGCUGAGAUAUGCCGCUUGGACAGGA GGCCUGGCUGCUGUGGUGCUGCUGUGUCUGGUGAUUUUUCUGAUCUGUACCGCC AAAAGGAUGAGAGUGAAAGCCGCCAGAGUGGAUAAGAGCCCCUAUAACCAGAGC AUGUACGGCGCCGGCCUGCCUGUGGAUGAUUUUGAGGAUGCCGAGGCUGCUGAU GCCGAAGAGGAGUUUGGCAAUGCCAUUGGCGGCAGCCACGGCGGCAGCAGCUAC ACCGUGUACAUCGACAAGACCAGA |
| 41 | VZVE-4-deltag-1-3 | AUGGGCACCGUGAAUAAGCCUGUGGUGGGCGUCCUGAUGGGAUUUGGAAUCAUC ACAGGCACCCUGAGGAUCACCAACCCCGUGAGAGCCAGCGUGCUGAGGUACGAU GACUUCCACAUCGACGAGGACAAGCUGGACACCAAUAGCGUGUACGAGCCCUAU UACCAUAGCGACCACGCCGAGAGCUCUUGGGUGAACAGAGGCGAGAGCAGCAGA AAGGCUUACGAUCACAACAGCCCUUAUAUCUGGCCCAGAAACGACUACGACGGC UUCCUGGAGAACGCCCACGAGCACCACGGCGUGUACAAUCAGGGAAGAGGCAUC GAUUCUGGAGAGCGACUGAUGCAGCCAACCCAGAUGUCUGCCCAGGAGGAUUUA GGCGAUGACACCGGAAUCCACGUGAUCCCUACCCUGAAUGGCGACGACAGACACA AGAUCGUGAAUGUGGAUCAGAGGCAGUACGGCGACGUGUUCAAGGGCGACCUGA ACCCAAAGCCACAGGGCCAGAGGCUGAUUGAAGUGAGCGUGGAGGAGAAUCACC CUUUUACCCUGAGGGCCCCAAUCCAGAGGAUUUAUGGCGUGAGGUAUACCGAGA CAUGGAGCUUCCUGCCUAGCCUGACAUGUACCGGCGAUGCCGCUCCAGCCAUCCA GCACAUCUGCCUGAAACACACCACCUGCUUUCAGGACGUGGUGGUGGAUGUUGA UUGCGCCGAGAACACCAAGGAAGAUCAGCUGGCCGAGAUCUCUUAUAGGUUCCA GGGCAAGAAAGAGGCCGACCAGCCAUGGAUCGUGGUGAAUACCAGCACACUGUU CGACGAGCUGGAGCUGGACCCACCUGAGAUUGAGCCAGGCGUGCUGAAAGUGCU GAGAACCGAGAAGCAGUACCUGGGAGUGUACAUCUGGAACAUGAGAGGCAGCGA CGGCACCUCUACCUACGCCACAUUUCUGGUGACCUGGAAGGGCGAUGAGAAAAC CAGGAAUCCCACACCUGCUGUGACACCCCAGCCUAGAGGCGCCGAGUUUCACAUG UGGAAUUACCACAGCCACGUGUUCAGCGUGGGCGACACAUUUAGCCUGGCCAUG CACCUGCAGUACAAGAUCCACGAGGCCCCAUUCGACCUGCUGUUAGAGUGGCUG UAUGUGCCUAUCGACCCUACAUGCCAGCCAAUGAGGCUGUACAGCACCUGUCUG UACCACCCAAAUGCCCCUCAGUGCCUGAGCCACAUGAACAGCGGAUGUACAUUCA CCUCUCCUCACCUGGCUCAGAGAGUGGCCAGCACAGUGUACCAGAAUUGUGAGC ACGCCGACAACUACACCGCCUAUUGCCUGGGCAUCAGCCACAUGGAGCCCUCUUU CGGCUUAAUUCUGCAUGACGGCGGCACCACACUGAAGUUUGUGGACACCCCCGA AUCUCUGUCUGGCCUGUACGUGUUCGUGGUGUAUUUUAACGGCCACGUGGAGGC CGUGGCCUACACAGUGGUGUCUACAGUGGAUCACUUCGUGAACGCCAUCGAGGA GAGAGGCUUUCCCCCUACAGCUGGACAGCCUCCUGCUACCACAAAACCUAAGGAG AUCACCCCCGUGAAUCCUGGCACAUCUCCCCUGCUGAGAUAUGCCGCUUGGACAG GAGGCCUGGCUGCUGUGGUGCUGCUGUGUCUGGUGAUUUUUCUGAUCUGUACCG CCAAAAGGAUGAGAGUGAAAGCCGCCAGAGUGGAUAAGAGCCCCUAUAAUCAGA GCAUGUACGGCGCCGGCCUGCCUGUGGAUGAUUUCGAGGAUGCCGAGGCUGCUG AUGCCGAAGAGGAGUUUGGCAAUGCCAUUGGCGGAAGCCACGGCGGCAGCAGCU ACACCGUGUACAUCGACAAGACCAGA |

## Claims

1. A VZV antigen variant, **characterized in that** the VZV antigen variant has a Y582G difference compared to the amino acid sequence shown in SEQ ID NO: 1;
and/or, the VZV antigen variant has a deletion of positions 561-623, 569-623, or 574-623 compared to the amino acid sequence shown in SEQ ID NO: 1;
and/or, the VZV antigen variant has a modification of the protein transmembrane domain and intracellular domain compared to the amino acid sequence shown in SEQ ID NO: 1, wherein the modification of the protein transmembrane domain and intracellular domain comprises replacing the transmembrane domain and intracellular domain of the VZV antigen with the transmembrane domain of the SARS-CoV-2 Spike protein or the transmembrane domain of the influenza H protein.

2. The VZV antigen variant according to claim 1, **characterized in that** the VZV antigen variant further has one or more of the following differences compared to the amino acid sequence shown in SEQ ID NO: 1: Y569A, S593A, S595A, T596A, and T598A; preferably, the VZV antigen variant further has all of the following differences compared to the amino acid sequence shown in SEQ ID NO: 1: Y569A, S593A, S595A, T596A, and T598A;
and/or, the modification of the protein transmembrane domain and intracellular domain comprises replacing the transmembrane domain and intracellular domain of the VZV antigen with the transmembrane domain of the SARS-CoV-2 Spike protein; the transmembrane domain and intracellular domain of the VZV antigen are positions 538-647 of SEQ ID NO: 1.

3. The VZV antigen variant according to claim 2, **characterized in that** the amino acid sequence of the transmembrane domain of the SARS-CoV-2 Spike protein is shown in SEQ ID NO: 10; the amino acid sequence of the transmembrane domain of the influenza H protein is shown in SEQ ID NO: 11.

4. The VZV antigen variant according to claim 1, **characterized in that** the amino acid sequence of the VZV antigen variant comprises an amino acid sequence as shown in any one of SEQ ID NOs: 2-9.

5. An isolated nucleic acid, **characterized in that** the isolated nucleic acid comprises a nucleotide sequence encoding the VZV antigen variant according to any one of claims 1-4.

6. The isolated nucleic acid according to claim 5, **characterized in that** the isolated nucleic acid is mRNA; preferably, the mRNA comprises one or more of a Cap1, a 5' UTR, and a 3' UTR-polyA; more preferably, the nucleotide sequence encoding the VZV antigen variant comprises a nucleotide sequence as shown in any one of SEQ ID NOs: 15-25, 27-36.

7. An expression element, **characterized in that** the expression element comprises a starting plasmid and the isolated nucleic acid according to claim 5 or 6; the starting plasmid is pCDNA3.1.

8. A method for preparing the isolated nucleic acid according to claim 6, **characterized in that** the method comprises in vitro transcription of the expression element according to claim 7.

9. A pharmaceutical composition, **characterized in that** the pharmaceutical composition comprises the VZV antigen variant according to any one of claims 1-4, the isolated nucleic acid according to claim 5 or 6, and the expression element according to claim 7, as well as a lipid;
preferably, the lipid is a composition consisting of an ionizable lipid compound, DSPC (1,2-distearoyl-sn-glycero-3-phosphocholine), cholesterol, and DMG-PEG2000 (1,2-dimyristoyl-rac-glycero-3-methoxypolyethylene glycol-2000);
more preferably, the pharmaceutical composition is a vaccine formulation; preferably, the pharmaceutical composition further comprises an adjuvant.

10. Use of the VZV antigen variant according to any one of claims 1-4, the isolated nucleic acid according to claim 5 or 6, or the pharmaceutical composition according to claim 9 in the preparation of a medicament for preventing VZV infection; preferably, the VZV infection is chickenpox or shingles, such as postherpetic neuralgia.
